# EUROPÄISCHE PATENTANMELDUNG

(11) **EP 0 878 491 A2**
(43) Veröffentlichungstag der Anmeldung: **18.11.1998**
(21) Anmeldenummer: 98105587.4
(22) Anmeldetag: 27.03.1998
(51) Int. Cl.: C08F 246/00, A61L 33/00, C07C 305/06

(54) **Heparinanaloge Homo- oder Copolymere, ihre Herstellung und Verwendung**

(30) Priorität: 15.05.1997 DE 19720369; 14.01.1998 DE 19801040
(71) Anmelder: HÜLS AKTIENGESELLSCHAFT, 45764 Marl (DE)
(72) Erfinder: Wulff, Günter, Prof. Dr., 40699 Erkrath (DE); Anders, Christine, Dr., 45721 Haltern (DE); Bellmann, Susanne, 40589 Düsseldorf (DE); Brock, Achim, 45475 Mülheim (DE)

(57) **Zusammenfassung**

Die Erfindung betrifft heparinanaloge Homo- oder Copolymere, enthaltend Repetiereinheiten der Formel I 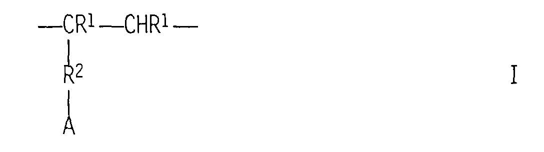 in der R¹ jeweils unabhängig Wasserstoff oder den Methylrest, R² ein Brückenglied und A einen sulfatierten Polyol-, Polyamin- oder (Poly)-amin(poly)ol-Rest bedeutet, der gegebenenfalls eine oder mehrere acetalisierte oder aminalisierte Carbonylfunktionen enthält: sowie ein Verfahren zu deren Herstellung. Die Erfindung betrifft weiter Sulfatgruppen-haltige und Sulfatgruppen-freie Monomere als Vorstufen der Homo- oder Copolymeren. Sie betrifft schließlich die Verwendung der heparinanalogen Homo- oder Copolymeren zur Beschichtung von Gegenständen für eine medizinische Verwendung und die so hergestellten Erzeugnisse.

## Beschreibung

Die Erfindung betrifft heparinanaloge, sulfatierte Monomere enthaltende Homo- oder Copolymere. Verfahren zur Herstellung dieser Homo-oder Copolymeren sowie deren Verwendung für medizinische Zwecke. Die Erfindung betrifft weiterhin die sulfatierten Monomere sowie deren Vorstufen als Zwischenprodukte für die Herstellung der heparinanalogen Polymeren.

### 1. Stand der Technik

Als Heparin bezeichnet man bekanntlich Glycosaminoglykane (oder Muco-polysaccharide) aus D-Glucosamin und D-Glucuronsäure mit einem Molgewicht von ca. 17.000, die O- und N-sulfatiert sind. D-Glucosamin und D-Glucuronsäure bilden jeweils 1,4-glycosidisch verknüpfte, disaccharidische Untereinheiten. die ihrerseits in einer dem Molekulargewicht entsprechender Anzahl 1,4-glycosidisch zum Heparin verknüpft sind. Heparin wirkt als Antikoagulans, verhindert also die Gerinnung des Blutes. In der Medizin findet Heparin Verwendung zur Therapie und zur Prophylaxe thromboembolischer Erkrankungen, zumeist in Form seines leicht wasserloslichen Natriumsalzes. Heparin wird oft adsorptiv auf Polymere aufgebracht. und derart "entgiftete" Polymere eignen sich für Gegenstände, die bei medizinischer Verwendung mit Blut in Berührung kommen, wie Herzklappen, Katheter, Endoskope und Drainageschlauche. Meist wird jedoch bei Operationen, bei denen Blut in Kontakt mit polymeren Werkstoffen kommt, prophylaktisch Heparin direkt dem Blut beigefügt.

### 2. Heparinanaloge Polymere nach der Erfindung

Erfindungsgemäß werden heparinanaloge Homo- oder Copolymere zur Verfügung gestellt, die Repetiereinheiten der Formel I 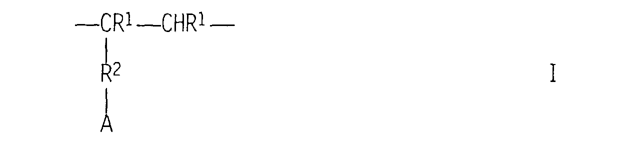 enthalten, in der
- R¹: jeweils unabhängig Wasserstoff oder den Methylrest,
- R²: ein Brückenglied und
- A: einen sulfatierten Polyol-, Polyamin- oder (Poly)amin(poly)-ol-Rest bedeutet, der gegebenenfalls eine oder mehrere acetalisierte oder aminalisierte Carbonylfunktionen enthält.

In den Repetiereinheiten der Formel I steht R¹ vorzugsweise für Wasserstoff.

Das Brückenglied R² kann anorganischer oder organischer Natur sein und steht bevorzugt für O, S, SO, SO₂ oder NR', wobei R' einen Kohlenwasserstoffrest mit 1 bis 12 Kohlenstoffatomen bezeichnet. für einen zweiwertigen organischen Rest, insbesondere für einen aliphatischen, cycloaliphatischen oder aromatischen Kohlenwasserstoffrest mit bis zu 10 Kohlenstoffatomen, eine Carbonesterbrücke -O-CO-, eine Carbonamidbrücke -NR' -CO- oder eine Urethanbrücke -O-CO-NR'-. wobei R' jeweils die angegebenen Bedeutung hat, oder für eine C-C-Einfachbindung.

Der Rest A leitet sich von einer Verbindung mit mindestens 2 Hydroxyl- und/oder Aminogruppen und vorzugsweise 2 bis 8. insbesondere 5 oder 6 Kohlenstoffatomen ab, enthält mindestens einen Rest -O-SO₃⁻M⁺ (O-Sulfatrest) oder -NH-SO₃⁻M⁺ (N-Sulfat- oder Amidosulfatrest), wobei M für ein Alkalimetallion, insbesondere für ein Natriumion steht. und enthält gegebenenfalls zusätzlich mindestens eine, vorzugsweise eine oder zwei Carbonylfunktionen, die unter Bildung eines Tetrahydrofuran- bzw. Pyrrolidinringes (mit jeweils 5 Ringgliedern) oder eines Pyran- bzw. Pentamethylenimin-Ringes (mit jeweils 6 Ringgliedern) intramolekular acetalisiert bzw, aminalisiert sind.

Zu den Verbindungen, von denen sich der Rest A ableiten kann. zählen u.a. Ethylenglykol, Ethylendiamin, Ethanolamin. Diethanolamin, Neopentylglykol. Glycerin. Glycerinaldehyd, Trimethylolpropan, Pentaerythrit, Erythrose. Erythrit, Threose, Threit, Heptanosen, Heptitole, Octanosen, Octitole sowie insbesondere Monosaccharide mit 5 oder 6 Kohlenstoffatomen (Pentosen oder Hexosen) und Aldehyd- oder Ketogruppen (Aldosen oder Ketosen) sowie die zugehörigen Zuckeralkohole (Pentitole oder Hexitole).

Die heparinanalogen Homo- oder Copolymere nach der Erfindung haben vorzugsweise ein Molekulargewicht von 5.000 bis 1.500.000, insbesondere von 50.000 bis 800. 000, bestimmt durch Membranosmometrie. Sie lassen sich verwenden wie Heparin und eignen sich besonders zur Beschichtung von Gegenständen aus Polymeren, die zur medizinischen Verwendung bestimmt sind. Sie zeigen teilweise eine stärkere antikoagulierende Wirkung als Heparin selbst. Die Thrombinzeit (TZ) und die Blutgerinnungszeit (PTT = partielle Thromboplastinzeit) sind daher verlängert. Zudem ist die antikoagulierende Wirkung der erfindungsgemäßen heparinanalogen Polymeren von längerer Dauer als die des Heparins. Offenbar werden die neuen Polymere als unphysiologische Stoffe von der Heparinase weniger leicht abgebaut, was auf nicht-glykosidischen Bindungen der Reste A an die Polymerkette zurückzuführen sein dürfte. Im Gegensatz dazu sind die glykosidischen Bindungen des Heparins leichter abbaubar.

Die heparinanalogen Homopolymere oder Copolymere können durch Polymerisation von Monomeren oder Comonomeren aufgebaut werden, die bereits die charakteristischen Gruppen R² und A enthalten. Alternativ kann man geeignete Polymere nachträglich zweckentsprechend verändern. So kann man Polymere mit (beispielsweise durch Ketalisierung mit Aceton) geschützten Repetiereinheiten entschützen und danach sulfatieren. Die Polymerisation der Monomeren mit den Gruppen R² und A ist das elegantere Verfahren, das zu besseren Ausbeuten und reineren Produkten führt, und wird daher bevorzugt. Die Herstellung der neuen Monomeren aus den Verbindungen, von denen sich der Rest A ableitet, erfolgt durch Reaktionen, die für sich bekannt sind und in der Folge erläutert werden. Die Herstellung verläuft zum Teil über eine ganze Reihe von Stufen, die jedoch durchweg gute Ausbeuten ergeben. Ein besonderer Vorteil besteht darin, daß man die Zwischenprodukte nicht zu reinigen braucht und auch die Monomere ohne irgendwelche Nachteile als Rohprodukte zu den erfindungsgemäßen Homo- oder Copolymeren polymerisieren kann.

### 3. Monomere für die neuen heparinanalogen Homo- oder Copolymere

Den Repetiereinheiten I der neuen heparinanlogen Homo- oder Copolymeren entsprechen die neuen Monomere der Formel II 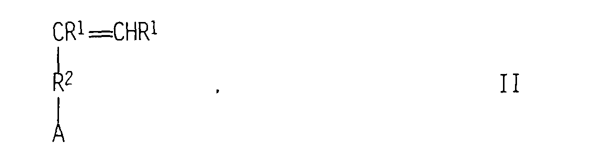 in der R¹, R² und A die für die Formel I angegebenen Bedeutungen einschließlich der bevorzugten Bedeutungen haben.

### 3.1 Von Pentitolen oder Hexitolen abgeleitete Monomere

Eine bevorzugte Klasse von Monomeren entspricht der (unter die Formel II fallenden) Formel III 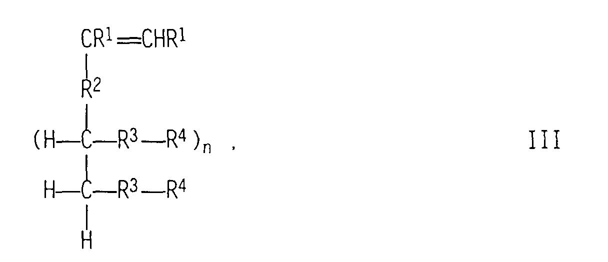 in der
- R¹ und R²: die zuvor angegebenen Bedeutungen einschließlich der bevorzugten Bedeutungen haben,
- R³: O oder NH,
- R⁴: Wasserstoff oder den Rest -SO₃⁻Na⁺ bedeutet und
- n: für 4 oder 5 steht;
mit der Maßgabe, daß mindestens einer der Substituenten R⁴ ein Rest -SO₃⁻Na⁺ ist.

In den Monomeren III bedeutet R² bevorzugt einen Alkylenrest mit 1 bis 4 Kohlenstoffatomen, einen Phenylenrest oder eine C-C-Einfachbindung.

Den Monomeren III entsprechen die Repetiereinheiten der Formel IIIa 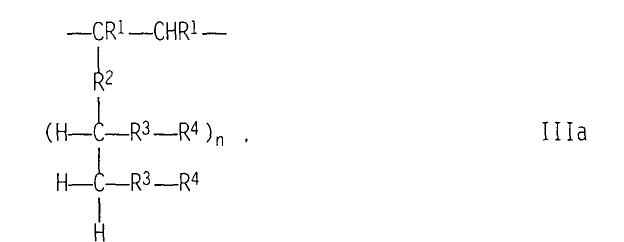 in der R¹, R², R³, R⁴ und n die für die Formel III angegebenen Bedeutungen einschließlich der bevorzugten Bedeutungen haben.

Durch die Formeln III und IIIa werden die Substituenten an der Kohlenstoffkette nicht stereochemisch festgelegt; die Darstellung entspricht also nicht einer Fischer-Projektion.

Die Monomere III (und die Repetiereinheiten IIIa) enthalten als Rest A (gemäß Formel II) von Pentitolen oder Hexitolen abgeleitete Gruppen, die wiederum auf Pentosen, wie Arabinose, oder auf Hexosen zurückgehen. Die Gruppen enthalten mindestens einen der Reste -O-SO₃⁻Na⁺ (O-Sulfat) oder -NH-SO₃⁻Na⁺ (N-Sulfat), bevorzugt benachbart zu dem Rest R². Sie können bis zu 5 (Pentitole) bzw. 6 (Hexitole) Sulfatreste enthalten und weisen vorzugsweise 1 bis 4 dieser Reste auf. In ein und derselben Gruppe können O-Sulfat- und N-Sulfatreste gleichzeitig vorliegen, wobei dann der N-Sulfatrest bevorzugt benachbart zu dem Rest R² positioniert ist. Alternativ kann die Gruppe aber auch ausschließlich eine Art dieser Reste enthalten, z.B. nur O-Sulfatreste.

### 3.1.1 Von Pentitolen abgeleitete Monomere III

Die Herstellung der Monomeren III wird anhand eines Spezialfalles beschrieben, der von D-Glucono-1,5-lacton 1 ausgeht und zu einem Monomer III führt, das von einer Pentose, nämlich der D-Arabinose, abgeleitet ist. Der Fachmann wird jedoch das Verfahren ohne weiteres auf andere geeignete Edukte übertragen können.

In einer ersten Stufe werden die Hydroxylgruppen des Lactons **1** durch Acetalisierung geschützt. z.B. mit Aceton, und wird gleichzeitig das (cyclische) Lacton mit Methanol zum (offenkettigen) Methylester umgeestert. Man erhält ein Isomerengemisch aus Methyl-3,4;5,6-di-O-iso-propyliden-D-gluconat **2** und Methyl-2,3;5,6-di-O-isopropyliden-D-gluconat **3.** Dieses Gemisch wird in einer **zweiten Stufe** reduziert, z.B. mit Lithiumaluminiumhydrid, wodurch die Carbonesterfunktion zur Carbinolfunktion wird. Man erhält wiederum ein Isomerengemisch, nämlich 3,4;5,6-Di-O-isopropyliden-D-sorbit **4** und 2,3;5,6-Di-O-isopropyliden-D-sorbit **5**. In einer dritten Stufe wird dieses Isomerengemisch mit einem Oxidationsmittel, wie Natriumperiodat, unter Spaltung der Kohlenstoffkette zu einem einheitlichen Produkt, dem Arabinosealdehyd 2,3;4,5-Di-O-isopropylidenaldehydo-D-arabinose **6** oxidiert. In der anschließenden **vierten Stufe** wird eine Vinylfunktion eingeführt, z.B. durch eine Grignard-Reaktion mit 4-Vinylphenylmagnesiumchlorid. Man erhält ein teilgeschütztes 4-Vinylphenylpentanpentaol, 2,3;4,5-Di-O-isopropyliden-1-(4-vinylphenyl)-D-gluco(D-manno)-pentitol **7**, das in der Folge kurz als Arasty bezeichnet wird.

Diese Stufenfolge 1 bis 4 wird durch das folgende Reaktionsschema veranschaulicht:

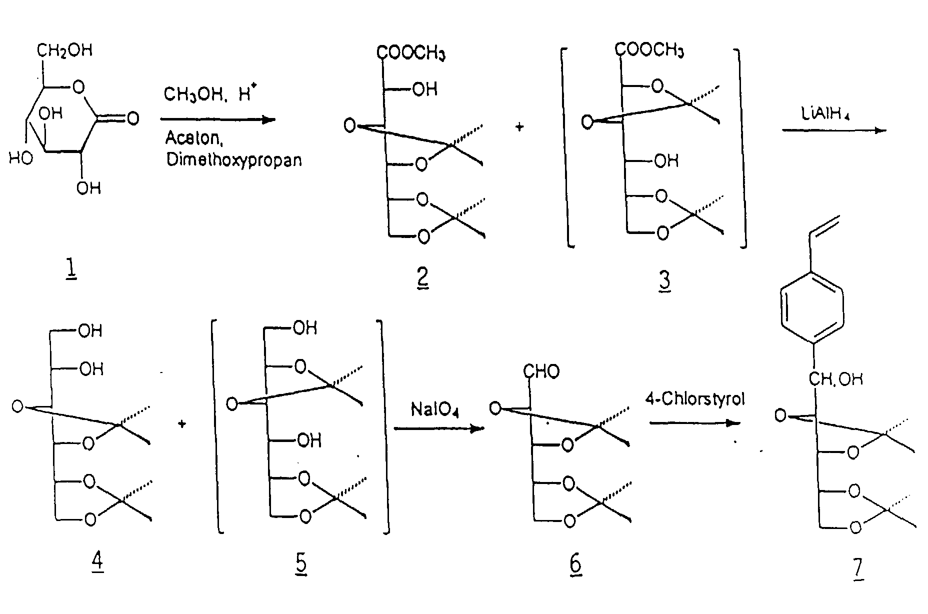

Die Reaktionsfolge der **Stufen 1** bis **3** (also bis zur Verbindung 6) wurde von H.Regeling et al. , Recl.Trav.Chim.Pays-Bas **1987**, (106) 461 und D.Y.Jackson, Synth.Commun. **1988** (18) 337 beschrieben. Die **Stufe 4** (zur Verbindung **7**) wurde von G.Wulff et al. , Macromol.Chem.Phys. **1996**, (197) 1285 erstmalig veröffentlicht.

Zur Herstellung einer dem Arasty **7** entsprechenden Verbindung mit einer Aminogruppe in 1-Stellung kann man Arasty in einer **ersten Stufe** zum entsprechenden Keton, (2,3;4,5-Di-O-isopropyliden-D-arabino)-(4-vinylphenyl)-keton **8.** oxidieren. Dieses wird in einer **zweiten Stufe** reduktiv in 1-Amino-1-desoxy-2,3;4,5-di-O-isopropyliden-1-(4-vinyl-phenyl)-D-gluco(D-manno)-pentitol **9** umgewandelt. Diese Reaktionsfolge wird durch das folgende Formelschema erläutert:

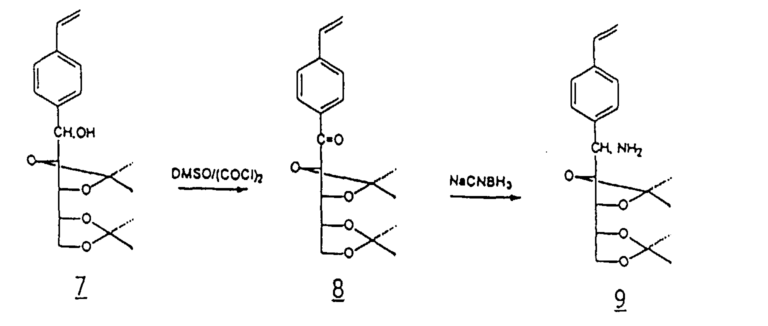

In der ersten Stufe kann Arasty **7** z.B. mit Oxalylchlorid und Dimethylsulfoxid bei einer Temperatur von <-50°C in einem inerten Lösemittel oxidiert werden. Die reduktive Aminierung in der zweiten Stufe erreicht man vorteilhaft mit Natriumcyanoborhydrid als Reduktionsmittel in Gegenwart von Ammoniumacetat in einem Lösemittel unter Wasserausschluß bei Raumtemperatur.

Heparin enthält ungeschützte Hydroxylgruppen und ist O-sulfatiert und N-sulfatiert. Die Verbindungen 7 und 9 werden daher in einer **ersten Stufe** entschützt (deacetalisiert) und in einer **zweiten Stufe** O- und/oder N-sulfatiert, damit das aus ihnen hergestellte Polymer möglichst weitgehend heparinanalog ist. Die Entschützung gelingt in saurem Medium, in dem Ketale nicht beständig sind. Man erhitzt die geschützten Verbindungen z.B. mit verdünnter Mineralsäure oder einem sauren Ionenaustauscher und erhält aus **7** 1-(4-Vinylphenyl)-D-gluco(D-manno)-pentitol **10** und aus **9** das 1-Anino-1-desoxy-1-(4-vinylphenyl)-D-gluco(Dmanno)-pentitol **11**. Die Entschützung und die nachfolgende Sulfatierung werden durch das folgende Formelschema wiedergegeben:

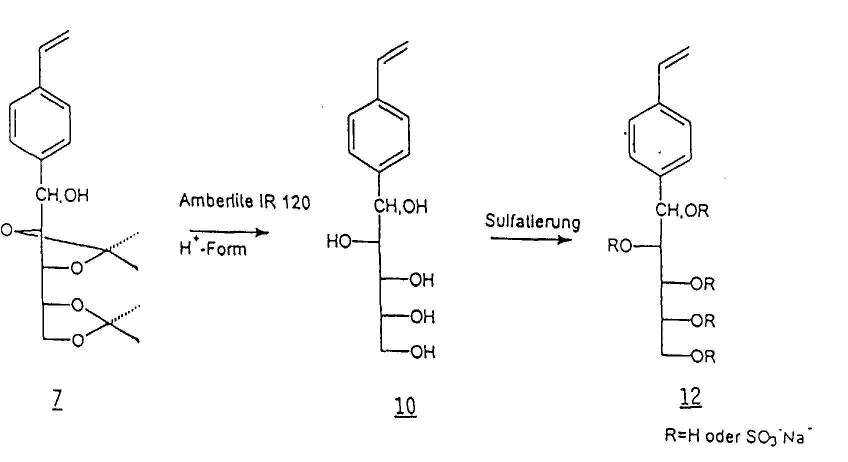

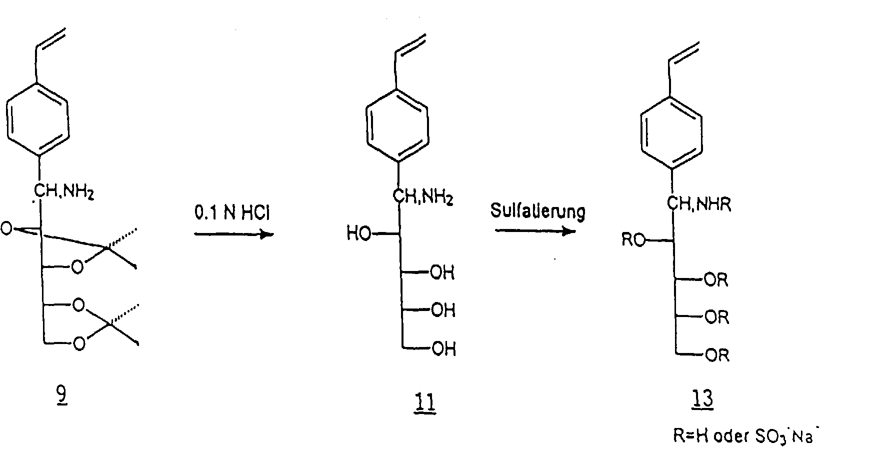

Die beiden Verbindungen **10** und **11** werden sulfatiert, zweckmäßig mittels eines Schwefeltrioxid-Pyridin-Komplexes. Wegen der vorweggenommenen Deacetalisierung kommt es bei der Sulfatierung nicht zu einem einheitlichen Produkt mit einer oder mehreren Sulfatgruppen in definierten Positionen. Es sollten jedoch die primären Hydroxylgruppen und die Aminogruppen bevorzugt sulfatiert werden. Durch Wahl eines geeigneten Molverhältnisses von Schwefeltrioxid zu Hydroxyl- bzw. Aminogruppen kann der Sulfatierungsgrad geregelt werden. Vorteilhaft wird durchschnittlich mehr als eine Sulfatgruppe pro Molekül eingeführt, da Heparin etwa 2,7 Sulfatgruppen pro Disaccharideinheit (entsprechend 1.35 Sulfatgruppen pro Molekül Monomer I) enthält. Durch Sulfatierung der entschützten Aminverbindung **11** erhält man gleichzeitig O-Sulfat- und N-Sulfatgruppen im Molekül, was im Hinblick auf die angestrebte Heparinanalogie erwünscht ist.

Die Sulfatierung wird vorteilhaft bei Raumtemperatur durchgeführt. um eine vorzeitige Polymerisation zu vermeiden. Durch längere Reaktions-dauer. z.B. bis zu 100 Stunden, kann die Reaktion trotzdem bis zum vollständigen Umsatz aller OH- bzw. NH₂-Gruppen geführt werden. Als Lösemittel kann man z.B. überschüssiges Pyridin oder einen Ether, wie Tetrahydrofuran, verwenden. Da die Sulfatgruppen der Reaktionsprodukte säurelabil sind, empfiehlt es sich, der Eduktlösung vor dem Zusatz des Schwefeltrioxid-Pyridin-Komplexes ein wasserbindendes Mittel zuzusetzen. z.B. ein Molekularsieb. Aus demselben Grunde ist es empfehlenswert. nach Beendigung der Reaktion das Reaktionsgemisch zunächst durch Zusatz von Wasser und bald darauf einer Base (die den pH im alkalischen Bereich hält) zu hydrolysieren. Eine geeignete Base ist z.B. eine gesättigte Bariumhydroxidlösung, die zugleich Sulfationen ausfällt. Überschüssige Bariumionen können z.B. durch Einleiten von Kohlendioxid gefällt werden, gegebenenfalls nach vorsichtigem Einengen unter Entfernung von Lösemittel. Das Bariumcarbonat wird abfiltriert und das Filtrat über eine Ionenaustauschersäule in der Na⁺⁻Form gegeben oder anderweitig mit dem Ionenaustauscher behandelt, um die Bariumionen gegen Natriumionen auszutauschen. Aus der weiter eingeengten Lösung kann man durch Gefriertrocknen die Produkte O-sulfatiertes 1-Hydroxy-1-desoxy-1-(4-vinylphenyl)-D-gluco(D-manno)-pentitol **12** bzw. N- und O-sulfatiertes 1-Amino-(4-vinylphenyl)-D-gluco(D-manno)-pentitol **13** jeweils in Form des Natriumsalzes als pulverförmige Feststoffe gewinnen.

### 3.1.2 Von Hexitolen abgeleitete Monomere III

Die Herstellung dieser Monomeren wird anhand eines weiteren Spezialfalles beschrieben, bei dem man wiederum von D-Glucono-1,5-lacton **1** ausgeht Und zwei Monomere erhält, die formal von einer Hexose, nämlich der D-Glucose, abgeleitet sind. Der Fachmann wird jedoch das Verfahren ohne weiteres auf andere geeignete Edukte übertragen und andere erfindungsgemäße Monomere herstellen können.

D-Glucono-1,5-lacton **1** wird zunächst in einer Eintopfreaktion mit einem sekundären Amin und Aceton in saurem Medium zu einem Regioisomerengemisch aus 3,4;5,6-Di-O-isopropyliden-D-gluconsäurediethylamid **14** und 2,3;5,6-Di-O-isopropyliden-D-gluconsäurediethylamid **15** umgesetzt. Aus diesem Gemisch ist durch Grignard-Reaktion mit Phenylmagnesiumchlorid ein Isomerengemisch aus 3,4;5,6-Di-O-isopropyliden-1-(4-vinylphenyl)-keto-D-glucose **16** und 2,3;5,6-Di-O-isopropyliden-1-(4-vinylphenyl)-keto-D-glucose **17** erhältlich.

Das Isomerengemisch **16** , **17** wird hydriert (oder reduziert), z.B. mit Natriumborhydrid oder Lithiumaluminiumhydrid, wodurch man zwei regioisomere Diastereomerenpaare, 3,4;5,6-Di-O-isopropyliden-1-(4-vinylphenyl)-D-glycero-D-gulo(D-ido)-hexitol und 2,3;5,6-Di-O-isopropyliden-1-(4-vinylphenyl)-D-glycero-D-gulo(D-ido)-hexitol **20** (im folgenden Formelschema nicht dargestellt) erhält. Diese können in saurem Medium entschützt werden, wodurch 1-(4-Vinylphenyl)-D-glycero-D-gulo(D-ido)-hexitol **21** entsteht. Durch Sulfatierung, z.B. mit dem Schwefeltrioxid-Pyridin-Komplex, wie beschrieben, erhält man hieraus das Sulfatierungsprodukt **22,** das ein Monomer III ist.

Alternativ kann man das Isomerengemisch **16** , **17** aminierend hydrieren (oder reduzieren), z.B. mit Natriumcyanoborhydrid und einem Ammoniumsalz. Man erhält dann zwei regioisomere Diastereomerenpaare. 1-Amino-1-desoxy-3,4;5,6-di -O-isopropyliden-1-(4-vinylphenyl )-D-glycero-D-gulo(D-ido)-hexitol und 1-Amino-1-desoxy-2,3;5,6-di-O-isopropyliden-1-(4-vinylphenyl)-D-glycero-D-gulo(D-ido)-hexitol **23** (im folgenden Formelschema nicht dargestellt). Diese können wiederum in saurem Medium entschützt werden, wodurch 1-Amino-1-desoxy-1-(4-vinylphenyl)-D-glycero-D-gulo(D-ido)-hexitol **24** erhalten wird, Dieses wird, wie beschrieben, zum Sulfatierungsprodukt **25** sulfatiert, das ebenfalls ein Monomer III ist.

Bei beiden Varianten kann die Reihenfolge der Hydrierung bzw. aminierenden Hydrierung und der Entschützung umgekehrt werden.

Die beschriebene Reaktionsfolge wird durch das folgende Formelschema wiedergegeben, wobei die Zwischenstufen **20** und **23** nicht dargestellt sind:

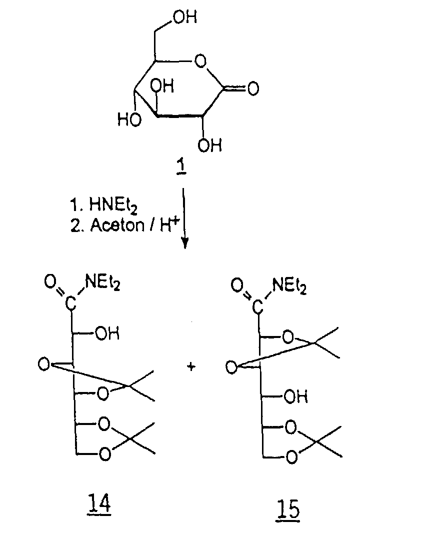

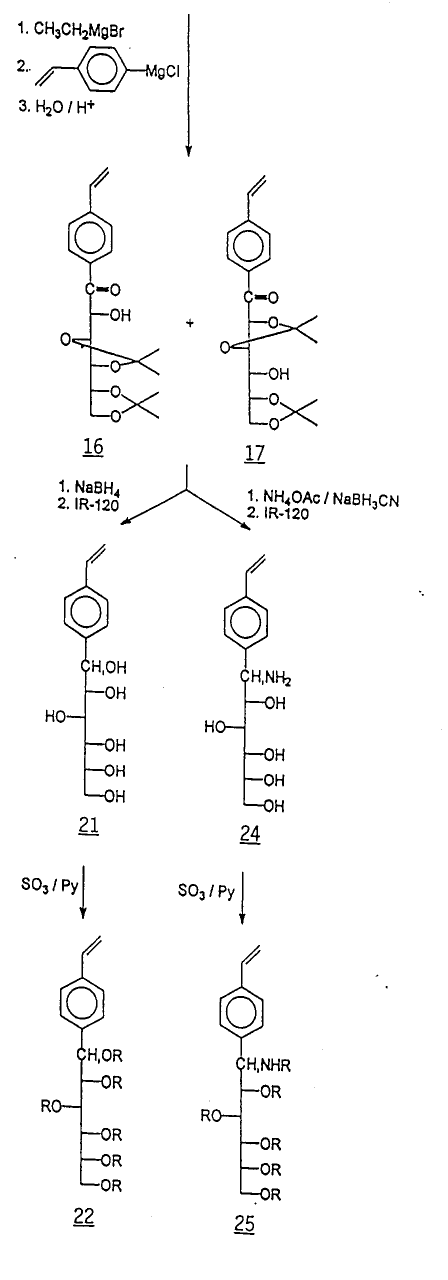

### 3.1.3 Monomere mit acetalisierter oder aminalisierter Carbonylfunktion

Bevorzugte Monomere dieser Art entsprechen der (unter die Formel II fallenden) Formel IV 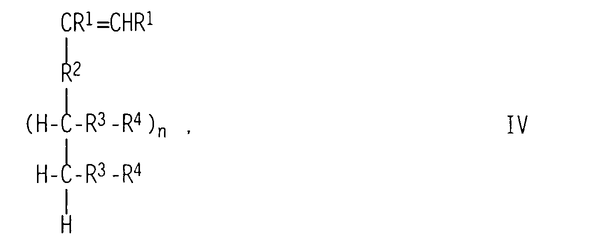
- in der: R¹, R², R³, R⁴ und n die für die Formel III angegebenen Bedeutungen einschließlich der bevorzugten Bedeutungen haben; mit der Maßgabe, daß
(1) mindestens einmal, vorteilhaft ein- oder zweimal pro Molekül an demselben Kohlenstoffatom stehende Substituenten H und -R³-R⁴ zusammen mit diesem Kohlenstoffatom eine Carbonylfunktion C=O bilden, die durch eine Hydroxyl- bzw. Aminofunktion in 3-Stellung in bezug auf die Carbonylfunktion unter Bildung eines Tetrahydrofuran- bzw. Pyrrolidinringes oder durch eine Hydroxyl- bzw. Aminofunktion in 4-Stellung in bezug auf die Carbonylfunktion unter Bildung eines Pyran- bzw. Pentamethyleniminringes intramolekular acetalisiert bzw. aminalisiert ist, und daß
(2) mindestens einer der Substituenten R⁴ ein Rest -SO₃⁻Na⁺ ist.

Den Monomeren IV entsprechen im (Co)polymer die Repetiereinheiten der Formel IVa 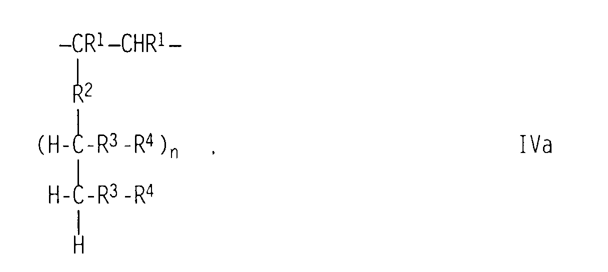
- in der: R¹, R², R³, R⁴ und n die für die Formel III angegebenen Bedeutungen einschließlich der bevorzugten Bedeutungen haben und die Maßgabebedingungen der Formel IV gelten.

Die Monomeren IV entsprechen prima facie auch der Formel III, sie unterscheiden sich aber von den Monomeren III durch die Maßgabebedingung (1). Die Monomeren IV leiten sich von Pentosen (n = 4) oder von Hexosen (n = 5) ab. Sie können sulfatierte Aldosen oder Ketosen sein, die beide in Form ihrer Acetale bzw. Aminale vorliegen. Die Formel IV gibt wiederum nicht die tatsächlichen stereochemischen Verhältnisse an der Kohlenstoffkette wieder, sondern umfaßt alle stereoisomeren Formen, in denen die Pentosen oder Hexosen vorkommen können, von denen sich die Monomeren IV ableiten.

Ein Monomer IV kann man z.B. herstellen. indem man zunächst verfährt. wie unter 3.1.2 beschrieben, aber aus dem Isomerengemisch **16**, **17** durch Entschützung in saurem Medium ein einheitliches Produkt, nämlich die 1-(4-Vinylphenyl)-keto-D-glucose **18**. herstellt. Diese wird in ihrer Acetalform durch Sulfatierung, wiederum z.B. mit Schwefeltrioxid/Pyridin. in die sulfatierte Verbindung **19** umgewandelt. die ein Monomer IV ist. Die Stufenfolge wird durch das folgende Formelschema wiedergegeben:

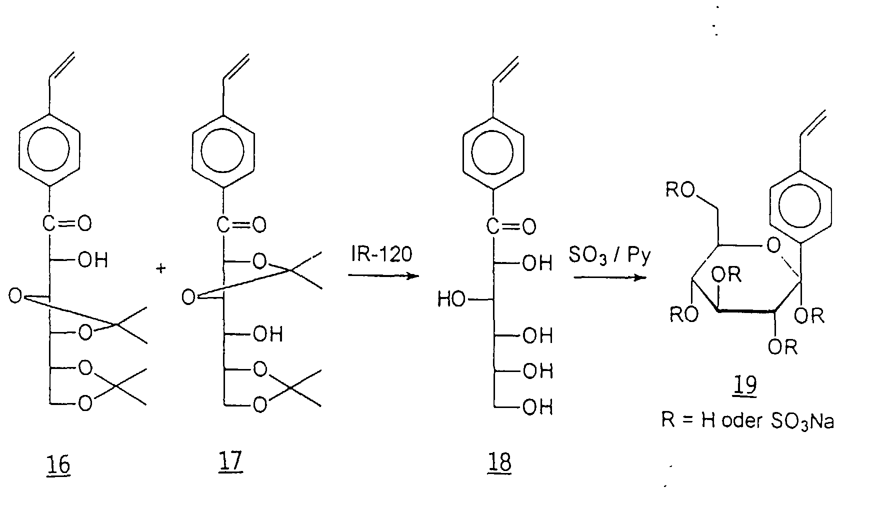

Andere Monomere IV nach der Erfindung lassen sich ausgehend von zunächst noch durch intermolekulare Acetalisierung (mit Aceton) geschützten und von Sulfatgruppen freien Monomeren herstellen. Die Herstellung eines von Sulfatgruppen freien ungeschützten Monomers, das sich von einer Ketohexose ableitet, sei am Beispiel der 1-(4-Vinylphenyl)-D-manno(D-gluco)-hexulo-2,6-pyranose **27** beschrieben. Man geht dazu aus von der Aceton-geschützten 2,3;4,5-Di-O-isopropyliden-1-(4-vinylphenyl)-D-manno(D-gluco)-hexulo-2,6-pyranose **26**. deren Herstellung von G.Wulff, J.Schmidt, T.P.Venhoff in Macrom.Chem.Phys., **197**, 1285 (1996) beschrieben wurde. Ein Beispiel für die Herstellung eines von Sulfatgruppen freien Monomers, das sich von einer Aldohexose ableitet, ist die Synthese der 6-(4-Vinylphenyl)-D-glycero(L-glycero)-α-D-galactopyranose **30**. Ausgangsstoff ist in diesem Falle die 1,2:3,4-Di-O-isopropyliden-6-(4-vinylphenyl)-D-glycero(L-glycero)-α-D-galaktopyranose **29**, deren Herstellung ebenfalls von G.Wulff, J.Schmidt, T.P.Venhoff a.a.O. beschrieben wurde. Die Entschützung der Hydroxylgruppen gelingt in beiden Fällen mittels eines sauren Ionenaustauschers in einem inerten Lösemittel. z.B. einem Alkohol. bei mäßig erhöhter Temperatur, wie 50 bis 100°C. zweckmäßig in einer Inertgasatmosphäre und in Gegenwart eines Oxidationsinhibitors.

Aus den noch von Sulfatgruppen freien Monomeren **27** und **30** (R⁴ jeweils H) kann man durch Sulfatierung deren vollständig oder teilweise sulfatierte (R = SO₃⁻Na⁺) Derivate herstellen. Man arbeitet beispielsweise wiederum mit einem Schwefeltrioxid-Pyridin-Komplex und zweckmäßig bei Raumtemperatur, um eine vorzeitige Polymerisation zu vermeiden. Nach längerer Zeit ist die Reaktion trotzdem vollständig. Der Sulfatierungsgrad kann durch Wahl eines geeigneten molaren Verhältnisses von Schwefeltrioxid zu Hydroxylgruppen bestimmt werden.

Die Herstellung der Monomeren IV **28** und **31** wird durch das folgende Formelschema beschrieben:

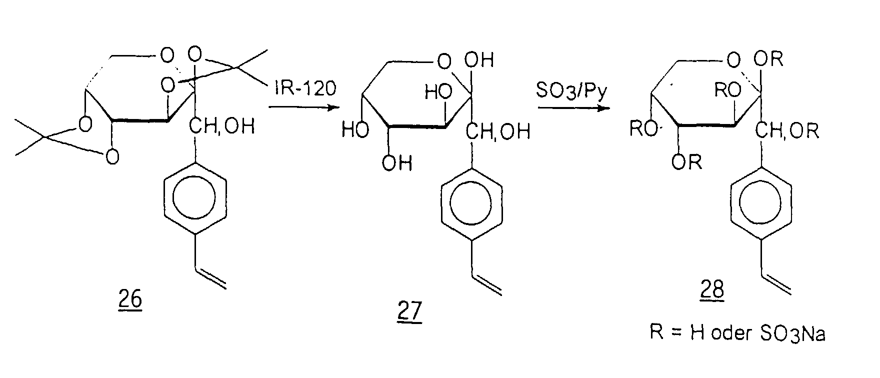

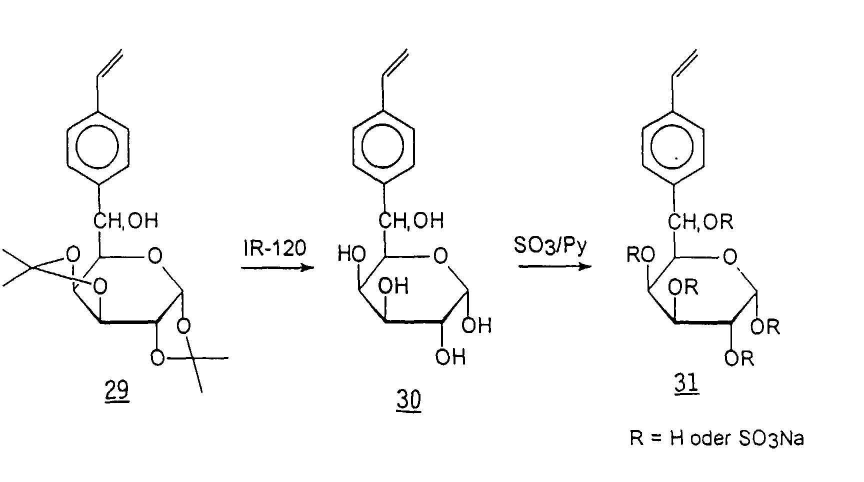

### 4. Neue Vorstufen der Monomeren II, III und IV

Auch die von Sulfatgruppen freien Vorstufen für die Sulfatgruppen enthaltenden Monomeren II, III und IV sind neue Stoffe, aus denen durch Sulfatierung, wie beschrieben, die Sulfatgruppen enthaltenden Monomere II, III und IV entstehen. Eine Ausnahme stellen 1-(4-Vinylphenyl)-D-gluco(D-manno)-pentitol (J.Schmid, Dissertation Universität Düsseldorf 1993; T.Venhoff, Dissertation Universität Düsseldorf 1993). 6-(4-Vinylphenyl)-D,L-glycero-α,β-D-galactopyranose (T.Venhoff, Dissertation Universität Düsseldorf 1993) 1-(4-Vinylphenyl)-D-manno(D-gluco)-hexulo-2,6-pyranose (H.Diederichs, Dissertation Universität Düsseldorf 1996) und 1-Vinyl-β-D-fructopyranose (T.Venhoff, Dissertation Universität Düsseldorf 1993) dar. Die neuen Vorstufen werden durch die folgenden Formeln wiedergegeben: 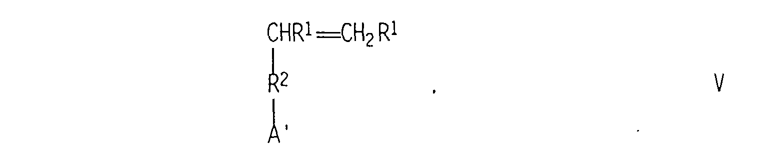
- in der: R¹ und R² die für die Formel II angegebenen Bedeutungen oder bevorzugten Bedeutungen haben und
A' einen nicht sulfatierten Polyol-, Polyamin- oder (Poly)amin(poly)ol-Rest bedeutet, der gegebenenfalls eine oder mehrere acetalisierte oder aminalisierte Carbonylfunktionen enthält; 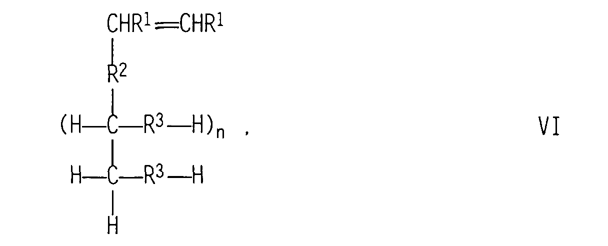
- in der: R¹, R², R³ und n die für Formel III angegebene Bedeutungen einschließlich der bevorzugten Bedeutungen haben; 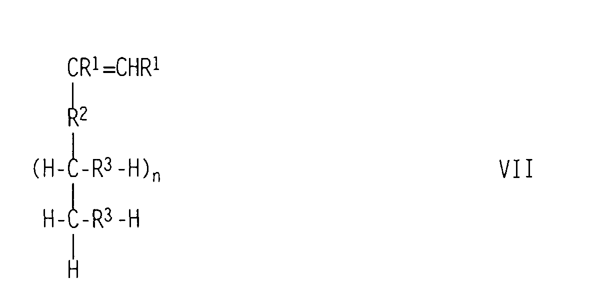
- in der: R¹ , R², R³ und n die für die Formel IV angegebene Bedeutung bzw. bevorzugte Bedeutung haben: mit der Maßgabe, daß mindestens einmal, vorteilhaft ein- oder zweimal pro Molekül an demselben Kohlenstoffatom stehende Substituenten H und -R³-H zusammen mit diesem Kohlenstoffatom eine Carbonylfunktion C=O bilden, die durch eine Hydroxyl- bzw. Aminofunktion in 3-Stellung in bezug auf die Carbonylfunktion unter Bildung eines Tetrahydrofuran- bzw. Pyrrolidinringes oder durch eine Hydroxyl- bzw. Aminofunktion in 4-Stellung in bezug auf die Carbonylfunktion unter Bildung eines Pyran- bzw. Pentamethyleniminringes intramolekular acetalisiert bzw. aminalisiert ist.

### 5. Herstellung der heparinanalogen Homo- oder Copolymeren

Die neuen heparinanalogen Polymere werden zweckmäßig durch Polymerisation oder Copolymerisation der Monomeren II, III und/oder IV hergestellt. Die Polymere können z.B. ausschließlich die Repetiereinheiten I. IIIa oder IVa als nur eine Spezies enthalten, z.B. als Repetiereinheit der Formel IIIa, die durchschnittlich 2,5 O-Sulfatreste trägt. In diesem Fall sowie in entsprechenden Fällen liegen Homopolymere vor. Die Repetiereinheit IIIa kann aber auch als Gemisch aus zwei oder mehr Spezies bestehen, beispielsweise aus der vorerwähnten Spezies und einer weiteren, die einen N-Sulfatrest in Nachbarschaft zu R² und dazu durchschnittlich 1 bis 3 O-Sulfatreste aufweist. In diesem Fall ist das Polymer ein Copolymer. Das Molverhältnis der verschiedenen Spezies kann in weiten Grenzen schwanken. Wenn z.B. die Repetiereinheit IIIa aus einer Spezies ausschließlich mit O-Sulfatresten sowie einer Spezies mit einem N-Sulfatrest und gegebenenfalls weiteren O-Sulfatresten besteht, kann es von 100:0 bis 0:100 betragen. Ein engerer. bewährter Bereich ist 20:1 bis 1:20.

Copolymere entstehen auch, wenn man ein oder mehrere Monomere II, III oder IV mit anderen, sulfatfreien Comonomeren V, VI, VII, VIII oder IX copolymerisiert.

Geeignete Comonomere VIII entsprechen der allgemeinen Formel VIII 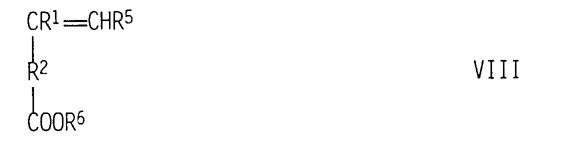 in der
- R¹ und R²: die für die Formel III angegebenen Bedeutungen einschließlich der bevorzugten Bedeutungen haben,
- R⁵: Wasserstoff, den Methylrest oder den Rest -R²-COOR⁶ und
- R⁶: Wasserstoff oder ein Alkalimetallion, insbesondere ein Natriumion bezeichnet.

Comonomere VIII führen zu Repetiereinheiten der Formel VIIIa 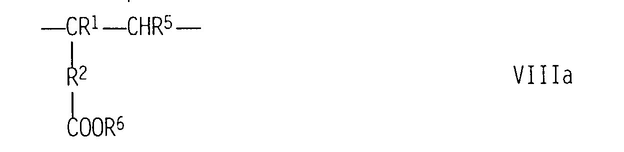 in der R¹, R², R⁵ und R⁶ die für die Formel VIII angegebenen Bedeutungen haben.

Die Comonomere VIII sind ethylenisch ungesättigte Mono- oder Dicarbonsäuren oder deren Carboxylate. d.h. deren Alkalisalze. insbesondere die Natriumsalze. Beispiele für geeignete Monomere VIII sind (Meth)acrylsäure, Crotonsäure, 4-Vinylbenzoesäure, Maleinsäure. Fumarsäure, Vinylsalicylsäure. Itaconsäure, Vinylessigsäure, Zimtsäure, 2- und 4-Vinylbenzoesäure, Methylmaleinsäure. Dimethylfumarsäure.

Methylfumarsäure. Dihydroxymaleinsäure und Allylessigsäure oder deren Natriumsalze. Anstelle der Comonomeren VIII kann man auch Derivate copolymerisieren, deren funktionelle Gruppen nach der Polymerisation in Carboxyl- oder Carboxylatgruppen umgewandelt werden können. Als solche Gruppen seien Aldehyd-, Carbonester-, Carbonsäureanhydrid- und Carbonitrilgruppen genannt. Comonomere VIII bzw. deren genannte Derivate sind bevorzugte Comonomere im Hinblick auf die heparinanalogen Eigenschaften der Copolymeren. Man kann wiederum verschiedene Spezies der Comonomeren VIII copolymerisieren, beispielsweise eine Mono- und eine Dicarbonsäure. Das Molverhältnis der Carboxyl- und/oder Carboxylatgruppen zu der Summe der O-Sulfat- und/oder N-Sulfatgruppen im Copolymer kann in weiten Grenzen schwanken, z.B. 100:1 bis 1:100 und vorteilhaft wiederum 20:1 bis 1:20 betragen.

Als Comonomere IX werden solche Vinylmonomere bezeichnet. die weder Sulfat- noch Carboxyl- oder Carboxylatgruppen enthalten und die Eigenschaften der heparinanalogen Copolymere in erwünschter Weise modifizieren. So können von hydrophilierenden Comonomeren IX oder von anderen Comonomeren IX stammende Repetiereinheiten IXa vorhanden sein, die die Verträglichkeit des heparinanalogen Copolymers mit einem zu beschichtenden Substratpolymer und damit dessen Haftung darauf verbessern. Von den geeigneten Comonomeren IX seien z.B. Ethylen, Propylen. 1-Buten, 1,3-Butadien, C₅- und C₆-Alkylene, Styrol, Vinyltoluol, (Meth)acrylnitril, (Meth)acrylamid, Acetylen, Acrolein, (Meth)acrylsäureester, Allylchlorid, Cinnamate, Crotonate, N-Vinylimidazol, Maleinsäureimid, N-Vinylpyrrolidin. N-Vinylpyrrolidon, N-Vinylsuccinimid, Vinylacetat, Vinylchlorid, Vinylmethyl-, -ethyl-und -butylether sowie Vinylmethyl- und -ethylketon genannt. Wenn weitere Repetiereinheiten vorhanden sind. beträgt deren Anteil in der Regel bis zu 90 Molprozent. insbesondere bis zu 70 Molprozent. bezogen auf auf die Summe der Repetiereinheiten I und II. Die obigen Angaben über das Molekulargewicht der heparinanalogen Polymeren und über das Molverhältnis der verschiedenen Spezies der Repetiereinheiten I, IIa, IIIa und/oder IVa sowie über das Verhältnis von Carboxyl-und/oder Carboxylatgruppen zu O-Sulfat- und/oder N-Sulfatgruppen gelten auch für Copolymere mit weiteren Repetiereinheiten.

Die Monomere II. III und/oder IV, gegebenenfalls auch zwei oder mehr Spezies von II, III und/oder IV, sowie gegebenenfalls Comonomere VIII und/oder IX lassen sich in an sich bekannter Weise polymerisieren. So kann man aus den Monomeren 22, 25 oder 31 ein heparinanaloges Homopolymer herstellen. Zur Herstellung eines Copolymers kann man z.B. als Monomer I die Verbindung **12** und als Monomer VIII Natriummethacrylat, Natriummaleat und/oder Natrium-4-vinylbenzoat einsetzen. Alternativ kann man auch von der Verbindung **13** ausgehen und dieselben Monomeren VIII verwenden. Sehr gute heparinanaloge Wirkungen erzielt man, wenn man die Verbindungen **12** und **13** mit jeweils durchschnittlich 1 bis 4 Sulfatgruppen pro Molekül und ein Monomer VIII, z.B. Natrium-4-vinylbenzoat, copolymerisiert. Wie zuvor erwähnt, können die Molverhältnisse der Carboxyl- und/oder Carboxylatgruppen zu den O-Sulfat-und/oder N-Sulfatgruppen in den genannten Monomeren bzw. in den entsprechenden Repetiereinheiten in weiten Grenzen schwanken und können z.B. 1:100 bis 100:1, vorteilhaft 20:1 bis 1:20 betragen. Auch das Molverhältnis von O-Sulfat- zu N-Sulfatgruppen ist. wie zuvor erwähnt, in weiten Grenzen variierbar und kann z.B. 0:100 bis 100:0 betragen. Auch hier ist ein engerer, bewährter Bereich 20:1 bis 1:20. Derartige Copolymere lassen sich durch die folgende Formel beschreiben:

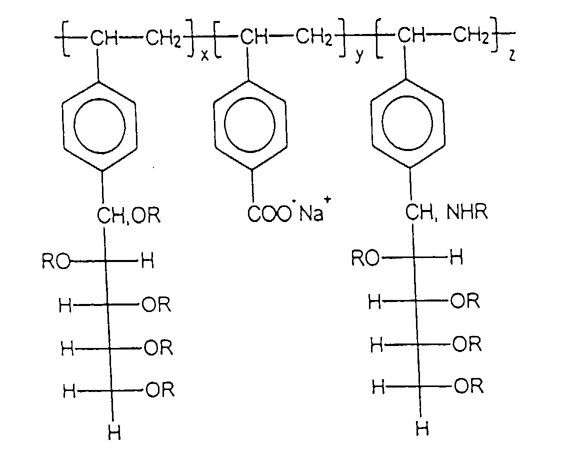

In der Formel bedeutet R Wasserstoff oder den Rest -SO₃⁻Na⁺, mit der Maßgabe. daß mindestens ein Rest R ein Rest -SO₃⁻Na⁺ ist. x und z bedeuten jeweils 0 oder ganze Zahlen, und y bezeichnet eine ganze Zahl. Die Copolymere haben vorzugsweise Molgewichte von 50.000 bis 800.000. bestimmt durch Membranosmometrie. Dementsprechend ist die Summe x+y+z, je nach dem Verhältnis der Repetiereinheiten. etwa 80 bis 1.100.

Die Polymerisation findet in wäßriger Lösung bei erhöhter Temperatur, z.ß. bei 60 bis 90°C. statt, Die Reaktionszeit schwankt erheblich, je nach Monomeren, Initiator, Initiatormenge und Temperatur, und beträgt in der Regel 5 bis 80 Stunden. Als Initiator hat sich 2,2'-Azo-bis(2-amidinopropan)dihydrochlorid besonders bewährt. Man wendet es vorteilhaft in Mengen von 0,5 bis 4 Molprozent an, bezogen auf die Summe der Monomeren I und II, um einen vollständigen Umsatz zu erreichen. Aus dem Reaktionsgemisch kann das Copolymer durch Zusatz von Alkohol ausgefällt und in üblicher Weise schonend von Wasser und Alkoholresten befreit werden.

### 6. Verwendung der heparinanalogen Copolymere

Die heparinanalogen Copolymere eignen sich hervorragend zur Beschichtung von Gegenständen, insbesondere von Gegenständen, die aus Polymeren bestehen und zur medizinischen Verwendung bestimmt sind, also mit Blut in Berührung kommen. Solche Gegenstände sind z.B. Herzklappen. Prothesen, Implantate, Katheter, Endoskope, Oxygenatoren. Dialysemembranen und Schläuche.

Die Beschichtung kann in üblicher Weise erfolgen, z.B. mit wäßrigen Lösungen, durch Tauchen, Streichen, Spritzen oder Spin-Coating. Durch zweckentsprechend Auswahl von Polymersubstrat und Copolymer kann man die Haftung des Copolymers auf dem Polymersubstrat günstig beeinflussen.

Zum besseren Verständnis der Erfindung werden die folgenden Beispiele gegeben, die jedoch den Anwendungsbereich der Erfindung nicht begrenzen sollen.

### 5. Beispiele

### 5.1 Herstellung von Methyl-3,4;5,6-di-O-isopropyliden-D-gluconat 2 und Methyl-2,3;5,6-di-O-isopropyliden-D-gluconat 3.

Eine Mischung aus 142,4 g (0,8 mol) D-Glucono-1,5-lacton **1.** 240 ml 2,2-Dimethoxypropan, 80 ml Aceton, 24 ml Methanol und 1,2 g p-Toluolsulfonsäure wird bei Raumtemperatur 48 h gerührt. Die erhaltene Mischung wird mit Natriumhydrogencarbonatlösung neutralisiert. Nach Filtration und Einengen des Filtrates nimmt man den resultierenden viskosen Sirup in etwa 500 ml Dichlormethan auf und wäscht ihn mit Wasser. Die Lösung wird eingeengt und der Rückstand im Ölpumpenvakuum destilliert. Ausbeute: 198 g (85%) Siedepunkt 112°C/0,04mbar, n_{D}²⁰: 1.455

### 5.2 Herstellung von 3,4;5,6-Di-O-isopropyliden-D-sorbit 4 und 2,3;5,6-Di-O-isopropyliden-D-sorbit 5.

In einem mit Stickstoff gespülten 4 l Dreihalskolben mit KPG-Rührer. Intensivkühler und Tropftrichter mit Blasenzähler werden 40 g LiAlH₄ in 1,3 l trockenem THF vorgelegt und 232 g (0,8 mol) der Ester 2 und 3 gelöst in 300 ml trockenem THF, langsam unter Kühlung durch ein Eis/Kochsalzbad zugetropft. Die Lösung wird 16 h unter Rückfluß erhitzt und nach dem Erkalten mit einer Mischung aus 400 ml 1,2-Dimethoxyethan und etwa 120 ml Eis (Tropftrichter) versetzt. Dann wird eine Mischung aus 150 ml Eis und 150 ml Wasser zugesetzt. Der entstehende Niederschlag wird abzentrifugiert, gründlich mit THF gewaschen und erneut zentrifugiert. Die vereinigten organischen Phasen werden am Rotationsverdampfer eingeengt, in Dichlormethan aufgenommen. mit 10 %-iger Ammoniumsulfat-Lösung und danach mit Natriumchlorid-Lösung gewaschen. Man trocknet die organische Phase über Natriumsulfat und engt sie am Rotationsverdampfer ein. Das Produkt wird im Vakuum fraktioniert destillert. Das Destillat erstarrt nach einiger Zeit zu weißen Kristallen. Ausbeute: 180 g (86%). Siedepunkt: 115-120°C/0,1 mbar. Smp.: 40-41°C

### 5.3 Herstellung von 2,3;4,5-Di-O-isopropyliden-aldehydo-D-arabinose 6

In einem 2 l Dreihalskolben mit KPG-Rührer, Rückflußkühler und Tropftrichter werden 131 g (0,5 mol) der beiden Diole **4** und **5** in 1.3 l Dichlormethan gelöst und unter kräftigem Rühren mit 160,4 g (0,75 mol) Natriummetaperiodat versetzt. Die Suspension wird 2 h bei 33-35°C kräftig gerührt. nachdem 50 ml gesättigte Natriumhydrogencarbonatlösung zugesetzt wurden. Dann gibt man 250 g Magnesiumsulfat zu und rührt weitere 15 min. Es wird filtriert und der Filterkuchen mehrmals gründlich mit Dichlormethan gewaschen. Die Filtrate werden vereinigt, das Lösemittel am Rotationsverdampfer entfernt und das Produkt fraktionierend destilliert.
Ausbeute: 86 g (75%). Siedepunkt: 85-90°C/0,1 mbar, n_{D}²⁰: 1,444

### 5.4 Herstellung von 2,3;4,5-Di-O-isopropyliden-1-(4-vinylphenyl). D-gluco(D-manno)-pentitol 7

Unter inerten Bedingungen (Stickstoffatmosphäre und Feuchtigkeitsausschluß) legt man in einem 1 l Dreihalskolben mit KPG-Rührer. Rückflußkühler und Tropftrichter 9,9 g (0,41 mol) Magnesium vor. 57 g (0,41 mol) 4-Chlorstyrol werden in 200 ml trockenem THF gelöst und nach dem Start der Reaktion mit etwas 1,2-Dibrommethan so zugetropft, daß das Reaktionsgemisch mäßig siedet. Nach beendeter Zugabe wird noch 2 h bei Raumtemperatur gerührt. Man kühlt das Reaktionsgemisch mit Hilfe eines Eis/Kochsalzbades auf unter 15°C. Dann werden 80 g (0,34 mol) des Aldehydes 6 in 200 ml trockenem THF so zugetropft, daß die Temperatur von 15°C nicht überschritten wird. Danach wird weitere 2 h bei Raumtemperatur gerührt. Man hydrolysiert durch Einschütten in Eiswasser. Die ausgefallenen Magnesiumsalze bringt man durch vorsichtige Zudosierung von 10 %iger Salzsäure in Lösung. Dabei soll ein pH-Wert von 4,5 nicht unterschritten werden. Die wäßrige Phase wird mit Ether extrahiert. Die vereinigten organischen Phasen werden mit Natriumhydrogencarbonatlösung neutralisiert und über Natriumsulfat getrocknet. Das Lösemittel wird am Rotationsverdampfer entfernt. Der resultierende Sirup kann säulenchromatographisch (Eluent A, Rf=0,40) gereinigt werden. Dabei werden vor allem das zuerst eluierende Styrol und entstandenes Polymer abgetrennt. Nach dieser Vorreinigung kristallisiert das Produkt im Kühlschrank aus und kann aus Petrolether/Diethylether umkristallisiert werden. Ausbeute 60-70%, Schmelzpunkt: 77°C (Diasteromer A) oder 59°C (Diasteromerengemisch A + B).

### 5.5 Herstellung von (2,3;4,5.Di-O-isopropyliden-D-arabino)-(4-vinylphenyl)-keton 8

Unter inerten Bedingungen legt man 15,7 ml (0,18 mol) Oxalylchlorid in 160 ml trockenem Dichlormethan vor und kühlt mittels eines Aceton/Trockeneisbades auf -60°C ab. Bei den nachfolgenden Reaktionen darf eine Temperatur von -50°C nicht überschritten werden. Unter kräftigem Rühren tropft man 15,3 ml (0.22 mol) Dimethylsulfoxid gelöst in 50 ml Dichlormethan zu. Nach beendeter Zugabe wird noch 15 min gerührt. Dann werden 50 g (0,15 mol) Arasty **7** in 150 ml Dichlormethan zugegeben. und es wird weitere 25 min gerührt. Zum Schluß gibt man 55,0 ml(0,39 mol) Triethylamin zu und rührt 10 min lang. Nach Erwärmen der Lösung auf Raumtemperatur wird der Reaktionsmischung das gleich Volumen Wasser zugesetzt. Die wäßrige Phase wird zweimal mit Dichlormethan extrahiert, und die vereinigten organischen Phasen werden nacheinander mit 10 % HCl. gesättigter NaHCO₃-Lösung und Wasser gewaschen. Man trocknet über Natriumsulfat und engt am Rotationsverdampfer ein. Die Reinigung des Rohproduktes erfolgt über Säulenchromatographie mit dem Eluenten A (R_{f}=0.53). Das Produkt fällt kristallin als weiße Nadeln an.
Ausbeute: 86%

| Elementaranalyse : C₁₉H₂₄O₅ 332,40 | | |
|---|---|---|
| | C(%) | H(%) |
| berechnet | 68,66 | 7,28 |
| gefunden | 68,75 | 7,14 |

### 5.6 Herstellung von 1-Amino-1-desoxy-2,3;4,5-di-O-isopropyliden-1-(4-vinylphenyl)-D-gluco(D-manno)-pentitol 9

In einem sekurierten 1 l Dreihalskolben mit Blasenzähler und N₂-Einleitung werden 20 g des Ketons **8** (60,2 mmol) und 60 g wasserfreies Ammoniumacetat (0,78 mol) vorgelegt, die in 600 ml trockenem Methanol gelöst werden. Um Wasserspuren zu beseitigen, gibt man etwas Molsieb 3 Å zu. Nach 30 Minuten Rühren bei Raumtemperatur werden im Stickstoffgegenstrom 3 g Natriumcyanoborhydrid (47,7 mmol) zugesetzt, und es wird 48 h bei Raumtemperatur gerührt. Die Reaktionslösung wird filtriert, auf ca. 200 ml eingeengt (33°C) und mit 200 ml destilliertem Wasser versetzt. Man stellt den pH-Wert der wäßrigen Lösung mit festem Kaliumhydroxid auf 10 ein und extrahiert mit Ether. Die organische Phase wird mit Magnesiumsulfat getrocknet, am Rotationsverdampfer eingeengt und säulenchromatographisch gereinigt (Eluent D, R_{f}=0,19 (Diastereomer A). R_{f} = 0,27 (Diastereomer B) oder Eluent E, R_{f}=0,16 (Diastereomer A), R_{f} = 0,21 (Diastereomer B)). Das Produkt ist ein gelbliches Öl.
Ausbeute: 11.58 g (57,7%)

| Elementaranalyse : C₁₉H₂₇O₄N 333,42 | | | |
|---|---|---|---|
| | C(%) | H(%) | N(%) |
| berechnet | 68,44 | 8,16 | 4,20 |
| gefunden | 68,31 | 8,17 | 4,06 |

### 5.8 Herstellung von 1-Amino-1-desoxy-1-(4-vinylphenyl)-D-gluco(D-manno)-pentitol 11

Man löst 1 g des Amins 9 (3 mmol) in 10 ml destilliertem Ethanol und setzt eine Spatelspitze Inhibitor (p-Methoxyphenol) zu. Dann wird langsam auf 80°C erhitzt und dabei tropfenweise insgesamt 60 ml (6 mmol) 0,1 n HCl zugesetzt. Nach 8 h bei 80°C läßt man abkühlen und extrahiert die wäßrige Lösung mit Diethylether. Die Lösung wird mit einem großen Überschuß Anionenaustauscher IRA 400 (OH⁻ -Form) gerührt und der Ionenaustauscher anschließend abfiltriert. Die Lösung ist danach stark alkalisch. Sie wird am Rotationsverdampfer auf ein kleines Volumen eingeengt (33°C) und gefriergetrocknet.
Ausbeute: 0,5 g (65,8%)

| Elementaranalyse : C₁₃H₁₉O₄N 253,30 | | | |
|---|---|---|---|
| | C(%) | H(%) | N(%) |
| berechnet | 61,64 | 7,56 | 5,53 |
| gefunden | 61,53 | 7,53 | 5,45 |

### 5.9 Herstellung von N- und O-sulfatiertem (Natriumsalz) 1-Amino-1-desoxy-1-(4-vinylphenyl)-D-gluco(D-manno)-pentitol 13.

In einem sekurierten 500 ml Dreihalskolben mit Blasenzähler und N₂-Einleitung werden 1 g (3,95 mmol) des entschützten Amins **11** in 60 ml Pyridin gelöst. und es wird etwas Molsieb (4 Å) zugesetzt. Nach 15 min Rühren bei Raumtemperatur setzt man 3,77 g (23,7 mmol) Schwefeltrioxid-Pyridin-Komplex zu und rührt 68 Stunden bei Raumtemperatur. Die Hydrolyse erfolgt zunächst mit 100 ml destilliertem Wasser und anschließend mit gesättigter Bariumhydroxidlösung, bis ein pH-Wert von ungefähr 9,5 erreicht ist. Das Pyridin wird azeotrop mit Wasser (35°C) entfernt. Dabei ist die Lösung ständig alkalisch. Der Überschuß an Bariumionen wird durch Einleiten von Kohlendioxid als Bariumcarbonat gefällt. Nach Zentrifugieren wird die Lösung mit einem großen Überschuß an Amberlite IR 120 (Na⁺-Form) gerührt. Nach Einengen am Rotationsverdampfer und Gefriertrocknung erhält man ein weißes Pulver.
Ausbeute: 84%

| Elementaranalyse : C₁₃H_{15,3}O_{15,1}NS_{3,7}Na 630,84 | | | | |
|---|---|---|---|---|
| | C(%) | H(%) | N(%) | S(%) |
| berechnet | 24,75 | 2,44 | 2,22 | 18,80 |
| gefunden | 24,45 | 2,46 | 2,00 | 19,20 |

### 5.10 Herstellung von 4-Vinylbenzoesäure

Unter inerten Bedingungen wird eine Lösung von 28 g (0.20 mol) 4-Chlorstyrol in 150 ml trockenem THF so zu 4,9 g (0.20 mol) Magnesiumspänen getropft, daß das Reaktionsgemisch gelinde siedet. Nach beendeter Zugabe läßt man unter Rühren auf Raumtemperatur erkalten.

Auf diese Lösung leitet man einen trockenen Kohlendioxidstrom. Dabei wird mit einem Eis/Kochsalzbad so gekühlt, daß die Innentemperatur 15°C nicht überschreitet. Wird kein Temperaturanstieg mehr festgestellt. leitet man weitere 30 min Kohlendioxid ein. Man hydrolysiert die Grignard-Lösung durch Eingießen in Eiswasser. Die Lösung wird vorsichtig mit 10 %iger Salzsäure angesäuert, bis schließlich ein pH-Wert von 1 erreicht ist. Die wäßrige Phase wird mit 200 ml Diethylether extrahiert, mit NaCl gesättigt und erneut dreimal mit je 100 ml Diethylether extrahiert. Die vereinigten organischen Phasen werden dreimal mit je 100 ml 10 %iger Natriumcarbonatlösung ausgeschüttelt und die vereinigen Sodaauszüge mit Diethylether gewaschen. Die wäßrige Phase wird mit konz. Salzsäure vorsichtig angesäuert. erneut mit NaCl gesättigt und erschöpfend mit Diethylether extrahiert. Die vereinigten organischen Phasen werden am Rotationsverdampfer bis zur Trockne eingeengt. Der resultierende Rückstand wird aus Ethanol/Wasser umkristallisiert.
Ausbeute: 16 g (52%), Schmelzpunkt 143°C

| Elementaranalyse : C₂₁H₂₈O₆ 376,45 | | |
|---|---|---|
| | C(%) | H(%) |
| berechnet | 67,00 | 7,50 |
| gefunden | 67,00 | 7,67 |

### 5.11 Herstellung von Natrium-4-vinylbenzoat

5.93 g 4-Vinylbenzoesäure (40 mmol) werden mit 39,9 ml einer 1 m NaOH-Lösung (39,9 mmol) versetzt. Die wäßrige Phase wird mit Ether extrahiert, um überschüssige 4-Vinylbenzoesäure zu entfernen. Die wäßrige Lösung wird gefriergetrocknet. Man erhält 6,79 g Natrium-4-vinylbenzoat als weißen Feststoff.

### 5.12 Herstellung eines Terpolymers aus den Monomeren 12. 13 und Natrium-4-vinylbenzoat

1 g (2 mmol) der deacetalisierten. O-sulfatierten (Natriumsalz) Verbindung **12**. 1 g (2 mmol) der deacetalisierten, N- und O-sulfatierten (Natriumsalz) Verbindung **13**. 0,35 g (2 mmol) Natrium-4-vinylbenzoat und 0,012 g ABAH (0,75 mol-%, bezogen auf alle eingesetzten Monomeren) werden in einem sekurierten 25 ml Einhalskolben vorgelegt, der mit einem Dreiweghahn zum Anschluß an die Vakuumlinie und Septum versehen ist. Bidestilliertes Wasser wird dreimal entgast. was durch Einfrieren und Auftauen der Lösung im Vakuum geschieht. Mit einer Hamiltonspritze entnimmt man im N₂-Gegenstrom 8 ml des entgasten Wassers und gibt dieses ebenfalls im Gegenstrom zu den Monomeren. Die Lösung wird unter Magnetrührung im Ölbad 72 h auf 80°C erhitzt. Nach beendeter Reaktion läßt man abkühlen und fällt das Polymer in 150 ml kaltem Ethanol aus. Das Polymer wird an der Ölpumpe mit Phosphorpentoxid getrocknet. Ausbeute: 75%

### 5.13 Bestimmung der heparinanalogen Wirksamkeit des Terpolymers 5.12 durch Vergleich der Blutgerinnungszeiten bei Zusatz des Terpolymers und von Heparin bei zwei Spenderplasmen

Die mit der partiellen Thrombplastinzeit (PTT) gemessene Blutgerinnungszeit wird durch gerinnungshemmende Stoffe, wie Heparin, beeinflußt. Zur Bestimmung der PTT wird Blutplasma mit einem Kontaktaktivator sowie Phospholipiden und Calciumionen versetzt. und damit eine Aktivierung der Kontaktaktivatoren ausgelöst sowie die Aktivierung des intrinsischen Gerinnungssystems katalysiert. Bei der praktischen Durchführung des Tests wird Blutplasma mit dem Kontaktaktivator (z.B. Kaolin) und Phospholipiden (z.B. Kephalin) versetzt und 3 Minuten bei 37°C inku biert. Dann wird Calciumchlorid zugesetzt und die Gerinnungszeit (PTT) gemessen. Zur Prüfung auf heparinanaloge Wirksamkeit wurde die PTT des Terpolymers mit der PTT von Heparin in Abhängigkeit von der Dosis verglichen. Die Dosen sind in I.E./ml angegeben. Die I.E. (Internationale Einheit des Heparins) ist diejenige. in der US-Pharmakopoe XXIII definierte Heparinmenge. die unter definierten Bedingungen die Umwandlung von 1 µmol Substrat/min katalysiert.

Die Thrombinzeit (TZ) ist ein weiteres Maß für die gerinnungshemmende Wirkung eines Stoffes. In der Endphase der Gerinnung wird aus Fibrinogen das unlösliche Fibrin, ein weiches Gerinnsel, das sich durch kovalente Querverbindung in hartes Gerinnsel umwandelt. Diese Reaktion wird durch Thrombin katalysiert und durch gerinnungshemmende Stoffe, wie Heparin. verzögert. Zur Bestimmung der heparinanalogen Wirksamkeit des Terpolymers wurde die TZ des Terpolymers mit der TZ von Heparin in Abhängigkeit von der Dosis verglichen. Die Dosen sind wiederum in I.E./ml angegeben.

Beim Vergleich der Gerinnungszeiten (PTT und TZ) des Terpolymers mit denen von Heparin wurde eine Standard-Heparinprobe (Molekulargewicht 13.000) mit 30 mg Heparin pro Milliliter verwendet, deren Gehalt nach Angaben des Herstellers 1.000 I.E pro Milliliter betrug. Das gemessene Molekulargewicht des Terpolymers von 149.000 (GPC/Lichtstreuung) wurde auf 100.000 abgerundet. da ein Fehler im Molekulargewicht nach unten hin zu einer kleineren Einwaage des Polymers führen und somit das Ergebnis des Gerinnungstests nur zuungunsten des Terpolymers beeinflussen kann. Ein als zu groß angenommenes Molekulargewicht würde dagegen ein stärkere Wirkung vortäuschen. Somit ergibt eine Einwaage von 0,23 g Terpolymer/ml einen Gehalt von ebenfalls 5.000 I.E./ml. Die Auswirkungen des Terpolymers auf die Blutgerinnungszeiten (PTT und TZ) im Vergleich zu Heparin wurden im Bereich zwischen 0,1 und 5 I.E. untersucht. Zur Durchführung des Vergleichstests wurden 9 Volumenteile Vollblut mit 1 Volumenteil entsprechend verdünnter Standardlösung des Heparins bzw. des Terpolymers gemischt. Die Gerinnungszeiten PTT und PT wurden nach Zentrifugieren des Blutes und Abtrennen des Plasmas bestimmt, wie zuvor beschrieben. Gerinnungszeiten über 10 Minuten wurden vom Koagulator (Firma Amelung, D-32657 Lemgo) in nicht erfaßt und wurden daher als > 600 s angegeben.

**Tabelle 1**

| Auswirkungen des Terpolymers aus 5.12 (TP) im Vergleich zu Heparin (HP) auf die PTT von zwei Spenderplasmen | | | | |
|---|---|---|---|---|
| Dosierung (I.E.) | PTT (sec) | | | |
| | Spender 1 | | Spender 2 | |
| | HP | TP | HP | TP |
| - | 34,5 | 33,9 | 31,9 | 33,3 |
| 0,01 | 35,5 | 30,9 | 34,3 | 30,7 |
| 0,05 | 46,0 | 32,6 | 46,0 | 37,3 |
| 0,1 | 68,5 | 36,7 | 68,0 | 43,3 |
| 0,2 | 109,7 | 53,5 | 97,1 | 54,6 |
| 0,3 | 155,5 | 84,6 | 104,5 | 67,9 |
| 0,4 | 179,7 | 157,3 | 123,9 | 109,0 |
| 0,5 | 248,8 | 209,3 | 184,5 | 128,9 |
| 1,0 | 574,6 | > 600 | 387,4 | > 600 |
| 5,0 | > 600 | | > 600 | |

**Tabelle 2**

| Auswirkungen des Terpolymers aus 5.12 (TP) im Vergleich zu Heparin (HP) auf die TZ von zwei Spenderplasmen | | | | |
|---|---|---|---|---|
| Dosierung (I.E.) | TZ (sec) | | | |
| | Spender 1 | | Spender 2 | |
| | HP | TP | HP | TP |
| - | 17,2 | 17,0 | 14,9 | 14,5 |
| 0,01 | 19,5 | 16,9 | 15,5 | 15,4 |
| 0,05 | 34,6 | 16,9 | 41,7 | 18,6 |
| 0,1 | 375 | 18,2 | 280,9 | 20,9 |
| 0,2 | > 600 | 21,9 | 425,8 | 26,1 |
| 0,3 | | 26,3 | > 600 | 37,1 |
| 0,4 | | 32,4 | | 39,2 |
| 0,5 | | 32,3 | | 39,2 |
| 1,0 | | 47,4 | | 56,4 |
| 5,0 | | > 600 | | > 600 |

Aus den Tabellen geht hervor, daß das Terpolymer im Vergleich zu Heparin schon bei geringerer Dosierung eine höhere PTT erreicht. Bei der Messung der TZ ergab sich, daß das Terpolymer erst ab 5,0 I.E. den Wert des Heparins bei 0,2 I.E. erreicht.

### 5.14 3,4:5,6-Di-O-isopropyliden-D-gluconsäurediethylamid 14 und 2,3:5,6-Di-O-isopropyliden-D-gluconsäurediethylamid 15

250 g D-Gluconolacton 1 werden in 500 ml Diethylamin suspendiert und über Nacht bei Raumtemperatur gerührt. Die Lösung wird im Vakuum eingeengt und der zähe Sirup vor dem Erstarren in eine Schüssel gegeben, um restliches Diethylamin von dem auskristallisierenden D-Gluconsäurediethylamid abdampfen zu lassen. 80 g des gelben Rohprodukts gibt man zu einer Mischung aus 1.500 ml Aceton und 35 ml konzentrierter Schwefelsäure und läßt 15 Stunden bei Raumtemperatur rühren. Zur Neutralisation wird der Ansatz unter Eiskühlung in 1.800 ml 1n Natronlauge gegossen, das Aceton am Rotationsverdampfer entfernt und die wäßrige Lösung erschöpfend mit Methylenchlorid extra-hiert. Die vereinigten organischen Phasen werden mit Wasser gewaschen, über wasserfreiem Natriumsulfat getrocknet und eingeengt. Das Rohprodukt wird im Ölpumpenvakuum fraktionierend destilliert. Die Ausbeute an dem Gemisch der beiden geschützten regioisomeren Amide **14, 15** in Form eines gelben Sirups beträgt 88 %.

### 5.15 2,3:5,6-Di-O-isopropyliden-1-(4-vinylphenyl)-keto-D-glucose 16 und 3,4:5,6-Di-O-isopropyliden-1-(4-vinylphenyl)-keto-D-glucose 17

In einer mit Stickstoff gespülten Apparatur werden 79,20 g (0,24 mol) der geschützten, regioisomeren Amide **14,** **15** in 120 ml trockenem Tetrahydrofuran vorgelegt. Bei einer Innentemperatur von <0°C werden 210 ml einer Im Ethylmagnesiumbromidlösung (0,21 mol) langsam zugetropft, bevor nach Temperaturerhöhung auf 15°C 460 ml einer Im Lösung von 4-Vinylphenylmagnesiumchlorid in Tetrahydrofuran (0,46 mol) zugegeben werden. Nach 3 Stunden Rühren bei Raumtemperatur wird der Ansatz durch vorsichtiges Zutropfen von 500 ml Wasser unter Eiskühlung hydrolysiert. mit konzentrierter Salzsäure auf pH 2 eingestelt und die organische Phase abgetrennt. Die wäßrige Phase wird dreimal mit Diethylether extrahiert. die vereinigten organischen Phasen werden mit gesättigter Natriumhydrogencarbonatlösung neutral gewaschen, über wasserfreiem Natriumsulfat getrocknet und bei einer Badtemperatur von <35°C eingeengt. Man erhält als Rohprodukt einen gelben Sirup in einer Ausbeute von 100%. Ein analysenreines Isomerengemisch **16,17** kann durch säulenchromatographische Feinreinigung im Eluenten Petrolether 40/60 und Diethylether (3:2) erhalten werden. Für die Nachfolgenden Reaktionen wird jedoch stets das Rohprodukt eingesetzt, ohne daß Nachteile für die Reinheit der Endprodukte festzustellen sind.

### 5.16 1- (4-Vinylphenyl)-keto-D-glucose 18

Zur Entschützung werden 25 g des Rohproduktes **16,** **17** in 300 ml destilliertes Ethanol eingetropft, und ausgefallene Polymeranteile werden abfiltriert. Das Filtrat wird innerhalb von 5 Stunden zu einem Gemisch von 1.300 ml Wasser, 200 ml destilliertem Ethanol, 20 g Ionenaustauscher Amberlite^{(R)}IR-120 (H⁺-Form) und 300 mg 4-Methoxyphenol bei 80°C langsam zugetropft. Nach einer weiteren Stunde wird der Ionenaustauscher abfiltriert, das Filtrat auf 150 ml eingeengt und zweimal mit Diethylether extrahiert. Durch Gefriertrocknung der wäßrigen Phase erhält man ein amorphes weißes Pulver in einer Ausbeute von 70 bis 75%, bezogen auf **16, 17**.

### 5.17 1-(4-Vinylphenyl)-D-glycero-D-gulo(D-ido)-hexitol 21

Man geht wiederum von dem Rohprodukt **16,** **17** aus. Zur Reduktion werden 10 g (max. 28 mmol) in 100 ml destilliertes Ethanol eingetropft und ausgefallene Polymeranteile werden abfiltriert. Zu dem eisgekühlten Filtrat wird eine Lösung von 0,76 g ( 20 mmol) Natriumborhydrid in 40 ml destilliertem Ethanol innerhalb von einer Stunde zugegeben. Nach weiteren 2 Stunden Rühren bei Raumtemperatur ist die Reduktion vollständig (Kontrolle durch DC im Laufmittel n-Hexan/Essigester 2:1). Die Reaktionslösung wird auf die Hälfte eingeengt, mit 100 ml Wasser versetzt und mit Diethylether extrahiert. Die vereinigten organischen Phasen werden mit Wasser gewaschen, über wasserfreiem Natriumsulfat getrocknet und eingeengt.

Der gelbe. sirupöse Rückstand wird in 150 ml destilliertem Ethanol aufgenommen und zu einem Gemisch von 500 ml Wasser, 80 ml destilliertem Ethanol, 8 g Ionenaustauscher Amberlite^{(R)}IR-120 (H⁺-Form) und 120 mg 4-Methoxvphenol innerhalb von 3 Stunden bei 80°C zugetropft. Das Gemisch wird eine Stunde weitergerührt. dann wird der Ionenaustauscher abfiltriert, das Filtrat auf ca. 150 ml eingeengt. zweimal mit Diethylether extrahiert und gefriergetrocknet. Das Produkt **21** fällt hierbei als amorphes, weißes Pulver an und kann aus Essigester oder Ethanol umkristallisiert werden. was sich aber lediglich auf das Diastereomerenverhältnis. nicht aber auf die Reinheit des Produkts auswirkt. Die Ausbeute beträgt 75 bis 80%. bezogen auf **16, 17**.

### 5.18 1-Amino-1-desoxy-1-(4-vinylphenyl)-D-glycero-D-gulo(D-ido)-hexitol 24

Zur reduktiven Aminierung werden 10 g des Rohprodukts **16,** **17** in 100 ml trockenes Methanol eingetropft und ausgefallene Polymeranteile abfiltriert. Das Filtrat wird unter Stickstoff vorgelegt und mit etwas Molsieb 3Å versetzt. Im Stickstoffgegenstrom werden 21,58 g (0,28 mol) Ammoniumacetat zugesetzt und nach einer Stunde Rühren bei Raumtemperatur 1,31 g (18,8 mmol) Natriumcyanoborhydrid (90%ig) hinzugefügt. Nach 48 Stunden wird der Ansatz filtriert, auf die Hälfte eingeengt, mit 100 ml Wasser versetzt und mit Diethylether extrahiert. Anschließend wird die wäßrige Phase mit festem Kaliumhydroxid auf pH 10 eingestellt und erneut mit Diethylether extrahiert. Die vereingten organischen Phasen werden mit Wasser gewaschen, über wasserfreiem Natriumsulfat getrocknet und eingeengt. Unverbrauchtes Edukt sowie Nebenprodukte werden mittels einer Filtriersäule (Rf>0,35, Fluent Essigster/n-Hexan/Triethylamin 80:20:1) abgetrennt.

Alle späteren Fraktionen werden eingeengt, in 150 ml destilliertem Ethanol aufgenommen und innerhalb von 4 Stunden zu einem Gemisch von 500 ml Wasser, 80 ml destilliertem Ethanol, 8 g Ionenaustauscher Amberlite^{(R)}IR-120 (H⁺-Form) und 120 mg 4-Methoxyphenol getropft. Eine Stunde nach beendeter Zugabe wird der Ionenaustauscher abfiltriert, das Filtrat auf ca. 150 ml eingeengt, zweimal mit Diethylether extrahiert und gefriergetrocknet. Das Produkt **24** fällt hierbei als amorphes, weißes Pulver an. Die Ausbeute beträgt bis zu 17% bezogen auf **16,** **17****.**

### 5.19 Sulfatierung von 18: 21; 24 / Standardvorschrift

In einem mit Stickstoff gespülten Kolben werden die entschützten Vinylmonomeren vorgelegt und durch Versetzen mit 40 ml trockenem Pyridin pro Gramm Vinylmonomer im Stickstoffgegenstrom gelöst. Nach Zusatz von etwas Mosieb 4Å wird eine halbe Stunde bei Raumtemperatur gerührt. bevor die dem gewünschten Sulfatierungsgrad entsprechende Moläquivalentmenge SO₃/Pyridin-Komplex (98%ig) zugegeben wird. Nach 24 Stunden wird die Reaktion durch Zusatz des halben Volumens von destilliertem Wasser beendet. Der Ansatz wird mit gesättigter Bariumhydroxyidlösung auf pH 7 eingestellt und filtriert. Aus dem Filtrat wird das Pyridin als Azeotrop mit Wasser bei <40°C entfernt, wobei der pH öfter kontrolliert und gegebenenfalls durch Zugabe von weiterer Bariumhydroxidlösung korrigiert wird. Überschüssige ßariumionen werden durch Einleiten eines CO₂-Gasstromes gefällt, und die Fällung wird abfiltriert. Dann wird die Lösung mit Ionenaustauscher Amberlite^{(R)}IR-120 (Na⁺-Form) gerührt, erneut filtriert, eingeengt und gefriergetrocknet. Die sulfatierten Produkte **22** und **25** fallen dabei als weiße, die sulfatierten Produkte **19** als gelbes Pulver an. Die Ausbeute beträgt 70 bis 95%. bezogen auf die jeweiligen Edukte.

### 5.20 Copolymere aus Monomeren II, III und/oder IV und Comonomer VIII Standardvorschrift

Die wasserlöslichen sulfatierten Monomeren II, III und/oder IV werden zusammen mit Natrium-4-vinylbenzoat als Comonomer VIII in einem Kolben mit aufgesetztem Dreiwegehahn und Septum zusammen mit dem Radikalstarter V50 (2,2'-Azobis-(2-amidinopropan)-dihydrochlorid) vorgelegt. ßidestilliertes Wasser wird dreimal entgast, was durch Einfrieren und Auftauen der Lösung im Vakuum geschieht. Mit einer gasdichten Spritze wird dieses Wasser über das Septum zugegeben. Die resultierende Lösung wird mit einem temperierten Ölbad auf 60°C erhitzt. Zum Abbruch der Polymerisation wird der abgekühlte Ansatz in das 15fache Volumen an kaltem Ethanol gegeben und das gefällte Polymer durch Zentrifugieren isoliert. Nach dem Trocknen an der Öl-pumpe über Phosphorpentoxid liegen die Copolymeren, die die Monomeren **22** und **25** enthalten, als weiße Pulver vor, diejenigen Monomer **19** besitzen eine gelb-bräunliche Farbe. Die Ausbeuten betragen 50 bis 98%.

### 5.21 Bestimmung der heparinartigen Wirksamkeit der Copolymeren aus 22, 25 und/oder 19 sowie Natrium-4-vinylbenzoat

Die heparinartige Wirksamkeit der Copolymeren wurde durch Vergleich der partiellen Thromboplastinzeiten (PTT) und der Thromboplastinzeiten (PT) bei Zusatz von Copolymer bzw, von standardisiertem Heparin geprüft, wie unter 5.13 beschrieben. Die erfindungsgemäßen Copolymeren zeigen in beiden Tests bessere Wirkungen als Standard-Heparin.

### 5.22 1-(4-Vinylphenyl)-D-manno(D-gluco)-hexulo-2,6-pyranose

Man aktiviert Ionenaustauscher Amberlite^{(R)}IR 120, indem man 10 g für 10 min mit 100 ml 2N HCl behandelt und nacheinander mit 100 ml destilliertem Wasser. 100 ml Methanol und wieder 100 ml destilliertem Wasser wäscht. Dann legt man in einem Kolben unter Stickstoff 10 g 2,3:4,5-Di-O-isopropyliden-1-(4-vinylphenyl)-D-manno(D-gluco)-hexulo-2,6-pyranose in 100 ml Ethanol vor und setzt 250 mg 4-Methoxyphenol zu. Unter Rühren werden langsam 50 ml Wasser zugefügt, und dann wird der aktivierte Ionenaustauscher zugesetzt. Zum Erhalt der körnigen Struktur des Ionenaustauschers wird ein Rührer mit Teflon^{(R)}-Blättern verwendet. Die Mischung im Kolben wird unter Rühren langsam erhitzt. Wenn die Temperatur 50°C erreicht, wird mit der tropfenweisen Zugabe von Wasser begonnen. Die Mischung im Kolben wird 18 bis 24 h auf 70 bis 80°C gehalten. Das Ausmaß der Entschützung wird durch Dünnschichtchromatographie kontrolliert. Als mobile Phase dient eine Mischung aus Petrolether (40-60°C) und Diethylether im Volumenverhältnis von 3:2. Nach dem Abkühlen der Mischung wird der Ionenaustauscher abfiltriert, und die wäßrige Lösung wird dreimal mit Diethylether extrahiert. Die wäßrige Lösung wird in einem Umlaufverdampfer im Vakuum bei 35 bis 38°C konzentriert. Durch Gefriertrocknung erhält das rohe Endprodukt als weißes Pulver. Durch Lösen in Ethanol und Filtrieren der Lösung in überschüssigen Diethylether wird das Reinprodukt in Form von weißen Kristallen vom Schmelzpunkt 161-163°C erhalten. [α]_{D} = -40,8°·dm⁻¹·g⁻¹N cm³ (c = 1,0 g/dl H₂O). IR (KBr): 3.520, 3.400 (OH) 3.180 (C=CH), 2.960, 2.920, 2.880 ((C-H), 1,630 (C=C), 1.510 cm⁻¹ (C=C arom.).

### 5.23 6-(4-Vinylphenyl)-D-glycero(L-glycero)-α-D-galactopyranose

Man verfährt wie in Beispiel 5.22, setzt aber als Ausgangsstof 15 g 1,2:3,4-Di-O-isopropyliden-6-(4-vinylphenyl)-D-glycero(L-glycero)-α-D-galaktopyranose ein. Man erhält das Produkt als weißes Pulver, das nicht umkristallisiert werden kann. Die Ausbeute beträgt 80 %. [α]_{D} = +45.1° deg dm⁻¹·g⁻¹·cm³ (c 1.0 g/dl H₂O). IR (KBr): 3.330 (OH), 2.900 (C-H). 1625 (C=C) und 1.510 cm⁻¹ (C=C arom.).

### 5.24 Sulfatierung von 1-(4-Vinylphenyl)-D-manno(D-gluco)-hexulo-2,6-pyranose

In einem sekurierten 500 ml Dreihalskolben mit Blasenzähler und Dreihalskolben wird 1 g (3,5 mmol) Ausgangsstoff in 60 ml Pyridin gelöst. und es wird etwas Molsieb A zugesetzt. Nach 15 min Rühren setzt man 1,69 g Schwefeltrioxid-Pyridin-Komplex zu und rührt .. Stunden bei Raumtemperatur. Die Hydrolyse erfolgt zunächst durch Zugabe von 100 ml destilliertem Wasser und anschließend mit gesättigter Bariumhydroxidlösung, bis ein pH von 9,5 erreicht ist. Das Pyridin wird als Azeotrop mit Wasser (35°C) entfernt, wobei die Lösung ständig alkalisch ist. Der Überschuß an Bariumionen wird durch Einleiten von Kohlendioxid als ßariumcarbonat gefällt. Nach dessen Abtrennung durch Zentrifugieren wird die Lösung mit einem großen Überschuß an Amberlite^{(R)}IR 120 in der Na⁺-Form gerührt. Nach Einengen am Rotationsverdampfer erhält man das Produkt als weißes Pulver.

### 5.13 Bestimmung der heparinanalogen Wirksamkeit eines sulfatierten Homopolymers

Das Homopolymer hatte die Formel 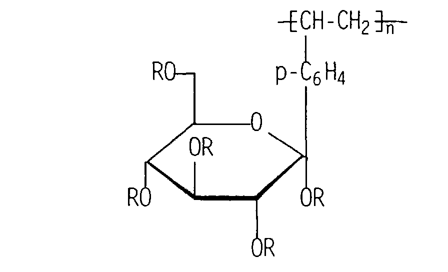 (R = H oder -SO₃Na, Sulfatierungsgrad 2,68; n entspricht einer durch Lichtstreuung ermittelten Molmasse von ca. 100.000). Die Messung der PTT nach Beispiel 5.13 ergab einen Wert von 63 sec bei Zusatz des Homopolymers (Dosierung 0.5 I.E./ml) gegenüber 30 sec PTT ohne diesen Zusatz. Das Homopolymer wirkte also antikoagulativ. Die Dosierung von 0.5 I.E./ml entspricht einer Dosierung von unfraktioniertem Heparin von 0,17 I.E./ml. Die antikoagulative Wirksamkeit ist also deutlich weniger ausgeprägt als die des Heparins.

## Patentansprüche

1. Heparinanaloge Homo- oder Copolymere, enthaltend Repetiereinheiten der Formel I 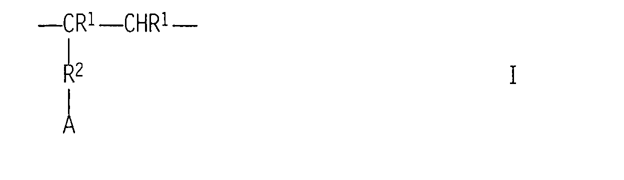 in der
R¹ jeweils unabhängig Wasserstoff oder den Methylrest,
R² ein Brückenglied und
A einen sulfatierten Polyol-, Polyamin- oder (Poly)amin(poly)-ol-Rest bedeutet, der gegebenenfalls eine oder mehrere acetalisierte oder aminalisierte Carbonylfunktionen enthält.

2. Polymere nach Anspruch 1, dadurch gekennzeichnet, daß
R¹ jeweils für Wasserstoff steht;
R² anorganischer oder organischer Natur ist und für O, S, SO, SO₂ oder NR'. wobei R' einen Kohlenwasserstoffrest mit 1 bis 12 Kohlenstoffatomen bezeichnet, für einen zweiwertigen organischen Rest, insbesondere für einen aliphatischen, cycloaliphatischen oder aromatischen Kohlenwasserstoffrest mit bis zu 10 Kohlenstoffatomen, für eine Carbonesterbrücke -O-CO-, eine Carbonamidbrücke -NR'-CO- oder eine Urethanbrücke -O-CO-NR'-, wobei R' die angegebenen Bedeutung hat. oder für eine C-C-Einfachbindung steht; und/oder
A einen einwertigen Rest bedeutet, der sich von einer Verbindung mit mindestens 2 Hydroxyl- und/oder Aminogruppen ableitet, die mindestens einen Rest -O-SO₃⁻M⁺ (O-Sulfatrest) oder -NH-SO₃⁻M⁺ (N-Sulfat- oder Amidosulfatrest) enthält, wobei M für ein Alkalimetallion steht, und gegebenenfalls zusätzlich mindestens eine Carbonylfunktion enthält. die unter Bildung eines Tetrahydrofuran- bzw. Pyrrolidinringes (mit jeweils 5 Ringgliedern) oder eines Pyran- bzw. Pentamethylenimin-Ringes (mit jeweils 6 Ringgliedern) intramolekular acetalisiert bzw. aminalisiert ist.

3. Polymere nach Anspruch 2, dadurch gekennzeichnet, daß R² einen aliphatischen, cycloaliphatischen oder aromatischen Kohlenwasserstoffrest mit bis zu 10 Kohlenstoffatomen bedeutet und/oder A sich von einer Verbindung ableitet, die 2 bis 8 Kohlenstoffatome enthält, in der M⁺ für ein Natriumion steht und die 1 oder 2 intramolekular acetalisierte oder aminalisierte Carbonylfunktionen enthält.

4. Polymere nach Anspruch 2 oder 3, dadurch gekennzeichnet, daß A sich von einer Verbindung ableitet, die 5 oder 6 Kohlenstoffatome enthält.

5. Polymere nach einem der Ansprüche 2 bis 4, dadurch gekennzeichnet, daß sie Repetiereinheiten der Formel IIIa 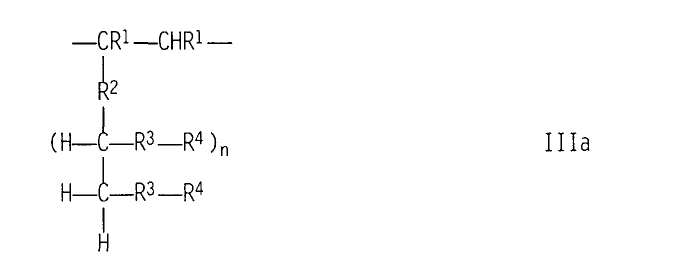 enthalten, in der
R¹ und R² die in den Ansprüchen 1 bis 4 angegebenen Bedeutungen haben,
R³ O oder NH.
R⁴ Wasserstoff oder den Rest -SO₃⁻Na⁺ bedeutet und
n für 4 oder 5 steht;
mit der Maßgabe, daß mindestens einer der Substituenten R⁴ ein Rest -SO₃⁻Na⁺ ist.

6. Polymere nach Anspruch 5, dadurch gekennzeichnet, daß sich die Repetiereinheiten von einem Pentitol ableiten.

7. Polymere nach Anspruch 5, dadurch gekennzeichnet, daß sich die Repetiereinheiten von einem Hexitol ableiten.

8. Polymere nach einem der Ansprüche 2 bis 4, dadurch gekennzeichnet. daß sie Repetiereinheiten der Formel 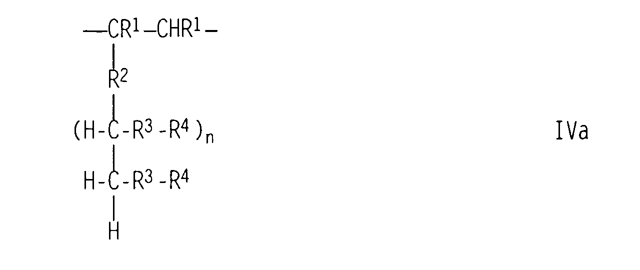 enthalten, in der
R¹, R², R³, R⁴ und n die in den Ansprüchen 1 bis 4 angegebenen Bedeutungen haben;
mit der Maßgabe, daß
(1) mindestens einmal pro Molekül an demselben Kohlenstoffatom stehende Substituenten H und -R³-R⁴ zusammen mit diesem Kohlenstoffatom eine Carbonylfunktion C=O bilden, die durch eine Hydroxyl- bzw. Aminofunktion in 3-Stellung in bezug auf die Carbonylfunktion unter Bildung eines Tetrahydrofuran-bzw. Pyrrolidinringes oder durch eine Hydroxyl- bzw. Amino-funktion in 4-Stellung in bezug auf die Carbonylfunktion unter Bildung eines Pyran- bzw. Pentamethyleniminringes intramolekular acetalisiert bzw. aminalisiert ist, und daß
(2) mindestens einer der Substituenten R⁴ ein Rest -SO₃⁻Na⁺ ist.

9. Polymere nach Anspruch 8, dadurch gekennzeichnet. daß die Repetiereinheiten 1 oder 2 intramolekular acetalisierte oder aminalisierte Carbonylfunktionen enthalten.

10. Polymere nach einem der Ansprüche 5 bis 9, dadurch gekennzeichnet, daß R² einen Alkylenrest mit 1 bis 4 Kohlenstoffatomen, einen Phenylenrest oder eine C-C-Einfachbindung bedeutet.

11. Polymere nach einem der Ansprüche 8 bis 10, dadurch gekennzeichnet, daß sich die Repetiereinheiten von einer Pentose ableiten.

12. Polymere nach einem der Ansprüche 8 bis 10, dadurch gekennzeichnet, daß sich die Repetiereinheiten von einer Hexose ableiten.

13. Polymere nach einem der Ansprüche 5 bis 12, dadurch gekennzeichnet, daß 1 bis 4 Substituenten R⁴ für -O-SO₃⁻Na⁺ (O-Sulfat) und/oder -NH-SO₃⁻Na⁺ (N-Sulfat) stehen.

14. Polymere nach einem der Ansprüche 1 bis 13, dadurch gekennzeichnet, daß sie Homopolymere mit nur einer, unter die Formeln I, IIIa oder IVa fallenden Repetiereinheit sind.

15. Polymere nach einem der Ansprüche 1 bis 13, dadurch gekennzeichnet, daß sie Copolymere aus mindestens zwei unter die Formeln I, IIIa oder IVa fallenden Repetiereinheiten sind.

16. Copolymere nach Anspruch 15, dadurch gekennzeichnet, daß sie eine Repetiereinheit IIIa oder IVa aufweisen, die nur O-Sulfatreste enthält, und eine andere Repetiereinheit IIIa oder IVa aufweisen, die einen N-Sulfatrest benachbart zu R enthält.

17. Copolymer nach Anspruch 15, dadurch gekennzeichnet, daß das Molverhältnis der beiden Spezies 1:100 bis 100:1 beträgt.

18. Copolymere nach einem der Ansprüche 15 bis 17, dadurch gekennzeichnet, daß sie Copolymere aus mindestens einer unter die Formeln I, IIIa oder IVa fallenden Repetiereinheit und mindestens einer weiteren Repetiereinheit der allgemeinen Formel VIIIa 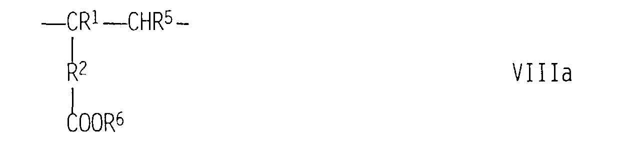 sind, in der
R¹ und R² die in den Ansprüchen 1 bis 4 angegebenen Bedeutungen haben.
R⁵ Wasserstoff, den Methylrest oder den Rest -R²-COOR⁶ und
R ⁶ Wasserstoff oder ein Natriumion bezeichnet.

19. Copolymer nach Anspruch 18, dadurch gekennzeichnet, daß das Molverhältnis der Carboxyl- und/oder Carboxylatgruppen in der oder den Repetiereinheit(en) VIIIa zu der Summe der O-Sulfat- und/oder N-Sulfatgruppen in der oder den Repetiereinheit(en) I. IIIa oder IVa 100:1 bis 1:100 beträgt.

20. Copolymere nach einem der Ansprüche 15 bis 19, dadurch gekennzeichnet, daß sie weiterhin mindestens eine Repetiereinheit aufweisen, die weder Sulfat- noch Carboxyl- oder Carboxylatgruppen enthält.

21. Homo- oder Copolymere nach einem der Ansprüche 1 bis 20, dadurch gekennzeichnet, daß ihr Molekulargewicht 5.000 bis 1.500.000 beträgt.

22. Homo- oder Copolymere nach Anspruch 21, dadurch gekennzeichnet, daß ihr Molekulargewicht 50.000 bis 800.000 beträgt.

23. Sulfatgruppen-haltige Monomere der Formel II 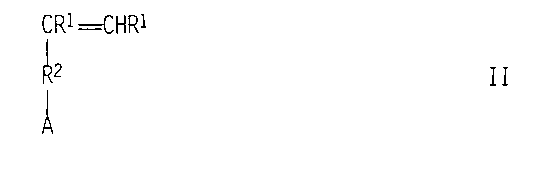 in der R¹, R² und A die in den Ansprüchen 1 bis 4 angegebenen Bedeutungen haben.

24. Sulfatgruppen-haltige Monomere nach Anspruch 23, dadurch gekennzeichnet, daß sie der unter die Formel II fallenden Formel III 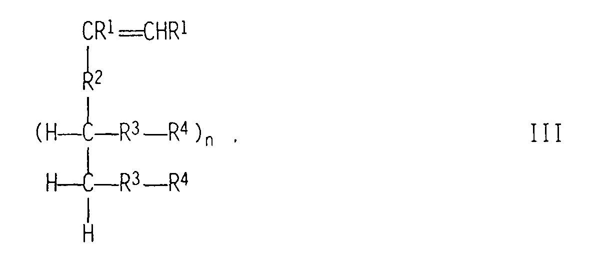 entsprechen. in der
R¹ und R² die in den Ansprüchen 1 bis 4 angegebenen Bedeutungen haben,
R³ O oder NH,
R⁴ Wasserstoff oder den Rest -SO₃⁻Na⁺ bedeutet und
n für 4 oder 5 steht;
mit der Maßgabe, daß mindestens einer der Substituenten R⁴ ein Rest -SO₃⁻Na⁺ ist.

25. Sulfatgruppen-haltige Monomere nach Anspruch 23, dadurch gekennzeichnet, daß sie der unter die Formel II fallenden Formel IVa 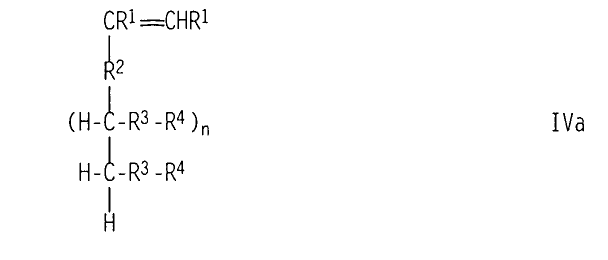 entsprechen, in der
R¹ , R², R³, R⁴ und n die in Anspruch 24 angegebenen Bedeutungen haben;
mit der Maßgabe, daß
(1) mindestens einmal pro Molekül an demselben Kohlenstoffatom stehende Substituenten H und -R³-R⁴ zusammen mit diesem Kohlenstoffatom eine Carbonylfunktion C=O bilden, die durch eine Hydroxyl- bzw. Aminofunktion in 3-Stellung in bezug auf die Carbonylfunktion unter Bildung eines Tetrahydrofuran-bzw. Pyrrolidinringes oder durch eine Hydroxyl- bzw. Amino-funktion in 4-Stellung in bezug auf die Carbonylfunktion unter Bildung eines Pyran- bzw. Pentamethyleniminringes intramolekular acetalisiert bzw. aminalisiert ist, und daß
(2) mindestens einer der Substituenten R⁴ ein Rest -SO₃⁻Na⁺ ist.

26. Sulfatgruppen-freie Monomere der Formel V. VI oder VII: 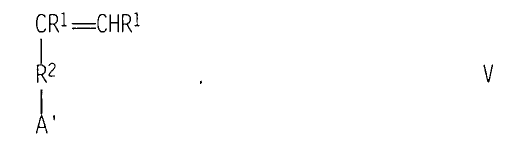
in der R¹ und R² die in den Ansprüchen 1 bis 4 angegebenen Bedeutungen haben und
A' einen nicht sulfatierten Polyol-, Polyamin- oder (Poly)-amin(poly)ol-Rest bedeutet, der gegebenenfalls eine oder mehrere acetalisierte oder aminalisierte Carbonylfunktionen enthält; 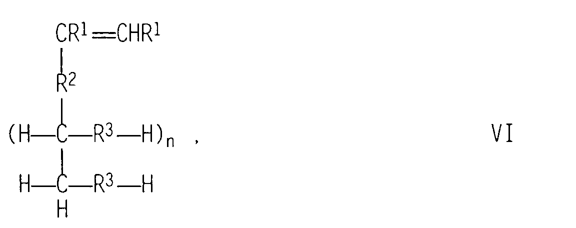
in der R¹, R², R³ und n die in Anspruch 24 angegebenen Bedeutungen haben; 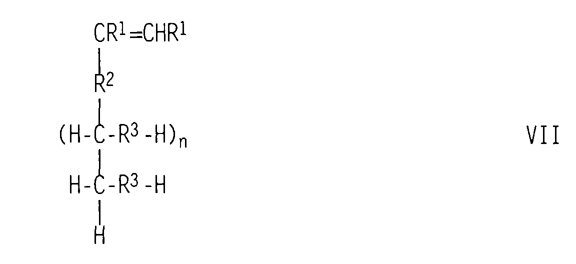
in der R¹, R², R³ und n die in Anspruch 25 angegebenen Bedeutungen haben; mit der Maßgabe, daß
mindestens einmal pro Molekül an demselben Kohlenstoffatom stehende Substituenten H und -R³-H zusammen mit diesem Kohlenstoffatom eine Carbonylfunktion C=O bilden, die durch eine Hydroxyl- bzw. Aminofunktion in 3-Stellung in bezug auf die Carbonylfunktion unter Bildung eines Tetrahydrofuran- bzw. Pyrrolidinringes oder durch eine Hydroxyl- bzw. Aminofunktion in 4-Stellung in bezug auf die Carbonylfunktion unter Bildung eines Pyran- bzw. Pentamethyleniminringes intramolekular acetalisiert bzw. aminalisiert ist;
wobei 1-(4-Vinylphenyl)-D-gluco(D-manno)-pentitol, 6-(4-Vinylphenyl)-D,L-glycero-α,β-D-galactopyranose 1-(4-Vinylphenyl)-D-manno(D-gluco)-hexulo-2,6-pyranose und 1-Vinyl-β-D-fructopyranose ausgenommen sind.

27. Verfahren zur Herstellung der heparinanalogen Homo- oder Copolymeren nach den Ansprüchen 1 bis 22, dadurch gekennzeichnet, daß man mindestens ein Sulfatgruppen-haltiges Monomer nach einem der Ansprüche 23 bis 25 und gegebenenfalls mindestens ein weiteres Monomer der allgemeinen Formel VIII 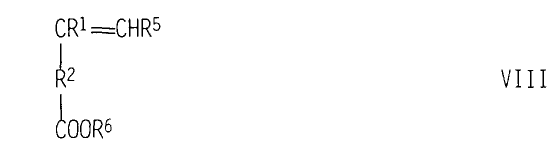 sind, in der R¹, R², R⁵ und R⁶ die in Anspruch 18 angegebenen Bedeutungen haben, und/oder gegebenenfalls mindestens ein Sulfat- und Carboxyl- oder Carboxylatgruppen-freies Vinylmonomer IX radikalisch initiiert copolymerisiert.

28. Verwendung der heparinanalogen Homo- oder Copolymeren nach einem der Ansprüche 1 bis 22 zur Beschichtung von Gegenständen für eine medizinische Verwendung.

29. Erzeugnisse für eine medizinische Verwendung, gekennzeichnet durch eine Beschichtung aus einem heparinanalogen Homo- oder Copolymer nach einem der Ansprüche 1 bis 22.

30. Erzeugnisse nach Anspruch 29, dadurch gekennzeichnet, daß die Erzeugnisse Herzklappen. Prothesen, Implantate, Katheter, Endoskope, Oxygenatoren, Dialysemembranen oder Schläuche sind.
